# EUROPEAN PATENT SPECIFICATION

(11) **EP 1 414 986 B1**
(45) Date of publication and mention of the grant of the patent: **01.10.2008**
(21) Application number: 02751164.1
(22) Date of filing: 30.07.2002
(51) Int. Cl.: C12P 13/08

(54) **CORYNEFORM BACTERIA WHICH PRODUCE CHEMICAL COMPOUNDS II**
CORYNEFORME BAKTERIEN, DIE CHEMISCHE SUBSTANZEN BILDEN II
BACTERIES CORYNEFORMES PRODUISANT DES COMPOSES CHIMIQUES II

(30) Priority: 06.08.2001 US 309877 P
(43) Date of publication of application: 06.05.2004
(73) Proprietor: Evonik Degussa GmbH, 45128 Essen (DE)
(72) Inventor: BATHE, Brigitte, 33154 Salzkotten (DE); REYNEN, Caroline, 33803 Steinhagen (DE); MÖCKEL, Bettina, 40597 Düsseldorf (DE); THIERBACH, Georg, 33613 Bielefeld (DE)
(86) International application number: PCT/EP2002/008465
(87) International publication number: WO 2003/014330

(56) References cited:
- EP-A- 1 108 790
- EP-A- 1 111 062
- WO-A-02/22632
- WO-A-99/18228
- US-B1- 6 200 785
- SCHAEFER A ET AL: "SMALL MOBILIZABLE MULTI-PURPOSE CLONING VECTORS DERIVED FROM THE ESCHERICHIA COLI PLASMIDS PK18 AND PK19: SELECTION OF DEFINED DELETIONS IN THE CHROMOSOME OF CORYNEBACTERIUM GLUTAMICUM" GENE, ELSEVIER BIOMEDICAL PRESS. AMSTERDAM, NL, vol. 145, no. 145, 1994, pages 69-73, XP001093898 ISSN: 0378-1119
- EIKMANNS B J ET AL: "MOLECULAR ASPECTS OF LYSINE, THREONINE, AND ISOLEUCINE BIOSYNTHESIS IN CORYNEBACTERIUM GLUTAMICUM" ANTONIE VAN LEEUWENHOEK, DORDRECHT, NL, vol. 64, no. 2, 1993, pages 145-163, XP000918559
- REINSCHEID ET AL.: "Stable expression of hom-1-thrB in Corynebacterium glutamicum" APPLIED AND ENVIRONMENTAL MICROBIOLOGY, vol. 60, no. 1, January 1994 (1994-01), pages 126-132,
- GÜNTHER KAHL: "The dictionary of gene technology" 2001, WILEY-VCH , WEINHEIM * page 327 * definitions of gene targeting and site-directed gene targeting * page 760 *
- SCHLEGEL: "Allgemeine Mikrobiologie" 1981, THIEM VERLAG , STUTTGART * page 450 - page 451 *

## Description

### Prior Art

Chemical compounds, which means, in particular, L-amino acids, vitamins, nucleosides and nucleotides and D-amino acids, are used in human medicine, in the pharmaceuticals industry, in cosmetics, in the foodstuffs industry and in animal nutrition.

Numerous of L-amino acids are prepared by fermentation from strains of coryneform bacteria, in particular Corynebacterium glutamicum. Because of their great importance, work is constantly being undertaken to improve the preparation processes. Improvements to the process can relate to fermentation measures, such as, for example, stirring and supply of oxygen, or the composition of the nutrient media, such as, for example, the sugar concentration during the fermentation, or the working up to the product form by, for example, ion exchange chromatography, or the intrinsic output properties of the microorganism itself.

Methods of mutagenesis, selection and mutant selection are used to improve the output properties of these microorganisms. Strains which are resistant to antimetabolites or are auxotrophic for metabolites of regulatory importance and which produce the particular compounds are obtained in this manner.

Methods of the recombinant DNA technique have also been employed for some years for improving the strain of Corynebacterium strains, by amplifying individual biosynthesis genes and investigating the effect on production.

A common method comprises amplification of certain biosynthesis genes in the particular microorganism by means of episomally replicating plasmids. This procedure has the disadvantage that during the fermentation, which in industrial processes is in general associated with numerous generations, the plasmids are lost spontaneously (segregational instability).

Schlegel: "Allgemeine Mikrobiologie" 1981, Thieme Verlag, Stuttgart, is an extract of a textbook, which discloses selection methods and methods for detecting different types of mutations.

Günter Kahl: "The dictionary of gene technology" 2001, Wiley-VCH, Weinheim, provides a definition of gene targeting and site-directed gene targeting.

Another method comprises duplicating certain biosynthesis genes by means of plasmids which do not replicate in the particular microorganism. In this method, the plasmid, including the cloned biosynthesis gene, is integrated into the chromosomal biosynthesis gene of the microorganism (Reinscheid et al., Applied and Environmental Microbiology 60(1), 126-132 (1994); Jetten et al., Applied Microbiology and Biotechnology 43(1):76-82 (1995).

Reinscheid discloses a vector and a method for introducing the hom-1-thrB operon of Corynebacterium glutamicum, which encodes a homoserine dehydrogenase resistant to feedback inhibition by L-threonine and homoserine kinase. It is shown that several copies of said operon can integrate into chromosome and that with increasing copy number the production of threonine increases. Furthermore, it is shown that the recombinant C. glutamicum is stable for more than 70 generations when grown in a medium without kanamycin.

A disadvantage of this method is that the nucleotide sequences of the plasmid and of the antibiotic resistance gene necessary for the selection remain in the microorganism. This is a disadvantage, for example, for the disposal and utilization of the biomass. Moreover, the expert expects such strains to be unstable as a result of disintegration by "Campbell type cross over" in a corresponding number of generations such as are usual in industrial fermentations.

### Object of the Invention

The inventors had the object of providing new measures for improved fermentative preparation of L-amino acids chosen from the group consisting of L-lysine, L-methionine, L-threonine and L-valine using coryneform bacteria.

### Summary of the Invention

The description provides coryneform bacteria, in particular of the genus Corynebacterium, which produce one or more desired chemical compounds, characterized in that
a) instead of the singular copy of an open reading frame (ORF), gene or allele naturally present at the particular desired site (locus), these have at least two copies of the said open reading frame (ORF), gene or allele, preferably in tandem arrangement, no nucleotide sequence which is capable of/enables episomal replication in microorganisms, no nucleotide sequence which is capable of/enables transposition and no nucleotide sequence which imparts resistance to antibiotics being present at the particular site, and in that these
b) optionally have at least a third copy of the open reading frame (ORF), gene or allele in question at a further gene site, no nucleotide sequence which is capable of/enables episomal replication in microorganisms, no nucleotide sequence which is capable of/enables transposition and no nucleotide sequence which imparts resistance to antibiotics being present at the further gene site.

The invention provides microorganisms as described in claims 1 to 13.

The decription also provides processes for the preparation of one or more chemical compounds, which comprise the following steps:
a) fermentation of coryneform bacteria, in particular of the genus Corynebacterium, which
   i) instead of the singular copy of an open reading frame (ORF), gene or allele naturally present at the particular desired site (locus), have at least two copies of the said open reading frame (ORF), gene or allele, preferably in tandem arrangement, no nucleotide sequence which is capable of/enables episomal replication in microorganisms, no nucleotide sequence which is capable of/enables transposition and no nucleotide sequence which imparts resistance to antibiotics being present at the particular site, and in that these
   ii) optionally have at least a third copy of the said open reading frame (ORF), gene or allele at a further gene site, no nucleotide sequence which is capable of/enables episomal replication in microorganisms, no nucleotide sequence which is capable of/enables transposition and no nucleotide sequence which imparts resistance to antibiotics being present at the further gene site,
   under conditions which allow expression of the said open reading frames (ORFs) genes or alleles,
b) concentration of the chemical compound(s) in the fermentation broth and/or in the cells of the bacteria,
c) isolation of the chemical compound(s), optionally
d) with constituents from the fermentation broth and/or the biomass to the extent of > (greater than) 0 to 100%.

The invention provides a process for the preparation of L-amino acids, chosen from the group consisting of L*-*lysine, L-methionine, L-threonine and L-valvine as described in claims 14 to 16.

### Detailed Description of the Invention

Chemical compounds are to be understood as meaning amino acids. The biosynthesis pathways of these compounds are known and are available in the prior art.

Amino acids mean L-amino acids, chosen from the group consisting of L-threonine, L-valine, L-methionine, L-lysine, and salts thereof. L-Lysine is very particularly preferred.

Proteinogenic amino acids are understood as meaning the amino acids which occur in natural proteins, that is to say in proteins of microorganisms, plants, animals and humans.

The coryneform bacteria are, in particular, those of the genus Corynebacterium. Of the genus Corynebacterium, the species Corynebacterium glutamicum, Corynebacterium ammoniagenes and Corynebacterium thermoaminogenes are preferred. Information on the taxonomic classification of strains of this group of bacteria is to be found, inter alia, in Kämpfer and Kroppenstedt (Canadian Journal of Microbiology 42, 989-1005 (1996)) and in US-A-5,250,434.

Suitable strains of the species Corynebacterium glutamicum (C. glutamicum) are, in particular, the known wild-type strains
Corynebacterium glutamicum ATCC13032
Corynebacterium acetoglutamicum ATCC15806
Corynebacterium acetoacidophilum ATCC13870
Corynebacterium lilium ATCC15990
Corynebacterium melassecola ATCC17965
Corynebacterium herculis ATCC13868
Arthrobacter sp ATCC243
Brevibacterium chang-fua ATCC14017
Brevibacterium flavum ATCC14067
Brevibacterium lactofermentum ATCC13869
Brevibacterium divaricatum ATCC14020
Brevibacterium taipei ATCC13744 and
Microbacterium ammoniaphilum ATCC21645
and mutants or strains, such as are known from the prior art, produced therefrom which produce said amino acids.

Suitable strains of the species Corynebacterium ammoniagenes (C. ammoniagenes) are, in particular, the known wild-type strains
Brevibacterium ammoniagenes ATCC6871
Brevibacterium ammoniagenes ATCC15137 and
Corynebacterium sp. ATCC21084
and mutants or strains, such as are known from the prior art, produced therefrom which produce said amino acids.

Suitable strains of the species Corynebacterium thermoaminogenes (C. thermoaminogenes) are, in particular, the known wild-type strains
Corynebacterium thermoaminogenes FERM BP-1539
Corynebacterium thermoaminogenes FERM BP-1540
Corynebacterium thermoaminogenes FERM BP-1541 and
Corynebacterium thermoaminogenes FERM BP-1542
and mutants or strains, such as are known from the prior art, produced therefrom which produce said amino acids.

Strains with the designation "ATCC" can be obtained from the American Type Culture Collection (Manassas, VA, USA). Strains with the designation "FERM" can be obtained from the National Institute of Advanced Industrial Science and Technology (AIST Tsukuba Central 6, 1-1-1 Higashi, Tsukuba Ibaraki, Japan). The strains of Corynebacterium thermoaminogenes mentioned (FERM BP-1539, FERM BP-1540, FERM BP-1541 and FERM BP-1542) are described in US-A 5,250,434.

Open reading frame (ORF) describes a section of a nucleotide sequence which codes or can code for a protein or polypeptide or ribonucleic acid to which no function can be assigned according to the prior art.

After assignment of a function to the nucleotide sequence section in question, it is in general referred to as a gene.

Alleles are in general understood as meaning alternative forms of a given gene. The forms are distinguished by differences in the nucleotide sequence.

In the context of the present invention, endogenous, that is to say species-characteristic, genes are preferably used. These are understood as meaning the genes or nucleotide sequences thereof present in the population of a species, such as, for example, Corynebacterium glutamicum.

A "singular copy of an open reading frame (ORF), gene or allele naturally present at the particular desired site (locus)" is understood as meaning the circumstances that a gene in general naturally occurs in one (1) copy in the form of its nucleotide sequence at its site or gene site in the corresponding wild-type or corresponding parent organism or starting organism. This site is preferably in the chromosome.

Thus, for example, the lysC gene or an lysC^{FBR} allele which codes for a "feed back" resistant aspartate kinase is present in one copy at the lysC site or lysC locus or lysC gene site and is flanked by the open reading frame orfX and the leuA gene on one side and by the asd gene on the other side.

"Feed back" resistant aspartokinases are understood as meaning aspartokinases which, compared with the wild-type form, have a lower sensitivity to inhibition by mixtures of lysine and threonine or mixtures of AEC (aminoethylcysteine) and threonine or lysine by itself or AEC by itself. Strains which produce L-lysine typically contain such "feed black" resistant or desensitized aspartokinases.

The nucleotide sequence of the chromosome of Corynebacterium glutamicum is known and can be found in the patent application EP-A-1108790 and Access Number (Accession No.) AX114121 of the nucleotide sequence databank of the European Molecular Biologies Laboratories (EMBL, Heidelberg, Germany and Cambridge, UK). The nucleotide sequences of orfx, the leuA gene and the asd gene have the Access Numbers AX120364 (orfX), AX123517 (leuA) and AX123519 (asd).

Further databanks, such as, for example, that of the National Center for Biotechnology Information (NCBI, Bethesda, MD, USA) or that of the Swiss Institute of Bioinformatics (Swissprot, Geneva, Switzerland) or that of the Protein Information Resource Database (PIR, Washington, DC, USA) can also be used.

"Tandem arrangement" of two or more copies of an open reading frame (ORF), gene or allele is referred to if these are arranged in a row directly adjacent in the same orientation.

"A further gene site" is understood as meaning a second gene site, the nucleotide sequence of which is different from the sequence of the ORF, gene or allele which has been at least duplicated at the natural site. This further gene site, or the nucleotide sequence present at the further gene site, is preferably in the chromosome and is in general not essential for growth and for production of the desired chemical compounds.

The "further gene sites" mentioned include, of course, not only the coding regions of the open reading frames or genes mentioned, but also the regions or nucleotide sequences lying upstream which are responsible for expression and regulation, such as, for example, ribosome binding sites, promoters, binding sites for regulatory proteins, binding sites for regulatory ribonucleic acids and attenuators. These regions in general lie in a range of 1-800, 1-600, 1-400, 1-200, 1-100 or 1-50 nucleotides upstream of the coding region. In the same way, regions lying downstream, such as, for example, transcription terminators, are also included. These regions in general lie in a range of 1-400, 1-200, 1-100, 1-50 or 1-25 nucleotides downstream of the coding region.

Intergenic regions in the chromosome, that is to say nucleotide sequences without a coding function, can furthermore be used. Finally, prophages or defective phages contained in the chromosome can be used for this.

A prophage is understood as meaning a bacteriophage, in particular the genome thereof, where this is replicated together with the genome of the host and the formation of infectious particles does not take place. A defective phage is understood as meaning a prophage, in particular the genome thereof, which, as a result of various mutations, has lost the ability to form so-called infectious particles. Defective phages are also called cryptic. Prophages and defective phages are often present in integrated form in the chromosome of their host. Further details exist in the prior art, for example in the textbook by Edward A. Birge (Bacterial and Bacteriophage Genetics, 3rd ed., Springer-Verlag, New York, USA, 1994) or in the textbook by S. Klaus et al. (Bakterienviren, Gustav Fischer Verlag, Jena, Germany, 1992).

To produce the coryneform bacteria according to the invention, the nucleotide sequence of the desired gene or allele, preferably including the expression and/or regulation signals, is isolated, at least two copies are arranged in a row, preferably in tandem arrangement, these are then transferred into the desired coryneform bacterium, preferably with the aid of vectors which do not replicate or replicate to only a limited extent in coryneform bacteria, and those bacteria in which two copies of the ORF, gene or allele are incorporated at the particular desired natural site instead of the singular copy originally present are isolated, no nucleotide sequence which is capable of/enables episomal replication in microorganisms, no nucleotide sequence which is capable of/enables transposition and no nucleotide sequence which imparts resistance to antibiotics remaining at the particular natural site (locus).

The expression and/or regulation signals mentioned, such as, for example, the ribosome binding sites, promoters, binding sites for regulatory proteins, binding sites for regulatory ribonucleic acids and attenuators lying upstream of the coding region of the ORF, gene or allele, are in general in a range of 1-800, 1-600, 1-400, 1-200, 1-100 or 1-50 nucleotides upstream of the coding region. The expression and/or regulation signals mentioned, such as, for example, the transcription terminators lying downstream of the coding region of the ORF, gene or allele, are in general in a range of 1-400, 1-200, 1-100, 1-50 or 1-25 nucleotides downstream of the coding region.

Preferably, also, no residues of sequences of the vectors used or species-foreign DNA, such as, for example, restriction cleavage sites, remain on the flanks of the ORFs, genes or alleles amplified according to the invention. In each case a maximum of 24, preferably a maximum of 12, particularly preferably a maximum of 6 nucleotides of such DNA optionally remain on the flanks.

At least a third copy of the open reading frame (ORF), gene or allele in question is optionally inserted at a further gene site, or several further gene sites, no nucleotide sequence which is capable of/enables episomal replication in microorganisms, no nucleotide sequence which is capable of/enables transposition and no nucleotide sequence which imparts resistance to antibiotics being present at the further gene site.

No residues of sequences of the vectors used or species-foreign DNA, such as, for example, restriction cleavage sites, remain at the further gene site. A maximum of 24, preferably a maximum of 12, particularly preferably a maximum of 6 nucleotides of such DNA upstream or downstream of the ORF, gene or allele incorporated optionally remain at the further gene site.

The description accordingly also provides a process for the production of coryneform bacteria which produce one or more chemical compounds, characterized in that
a) the nucleotide sequence of a desired ORF, gene or allele, preferably including the expression and/or regulation signals, is isolated
b) at least two copies of the nucleotide sequence of the ORF, gene or allele are arranged in a row, preferably in tandem arrangement
c) the nucleotide sequence obtained according to b) is incorporated in a vector which does not replicate or replicates to only a limited extent in coryneform bacteria,
d) the nucleotide sequence according to b) or c) is transferred into coryneform bacteria, and
e) coryneform bacteria which have at least two copies of the desired ORF, gene or allele at the particular desired natural site instead of the singular copy of the ORF, gene or allele originally present are isolated, no nucleotide sequence which is capable of/enables episomal replication in microorganisms, no nucleotide sequence which is capable of/enables transposition and no nucleotide sequence which imparts resistance to antibiotics remaining at the particular natural site (locus), and
f) at least a third copy of the open reading frame (ORF), gene or allele in question is optionally introduced at a further gene site, no nucleotide sequence which is capable of/enables episomal replication in microorganisms, no nucleotide sequence which is capable of/enables transposition and no nucleotide sequence which imparts resistance to antibiotics remaining at the further gene site.

The invention provides a process described in claims 13 to 16.

By the measures according to the invention, the productivity of the coryneform bacteria or of the fermentative processes for the preparation of chemical compounds is improved in respect of one or more of the features chosen from the group consisting of concentration (L-amino acid formed, based on the unit volume), yield (L-amino acid formed, based on the source of carbon consumed) and product formation rate (L-amino acids formed, based on the time) by at least 0.5 - 1.0% or at least 1.0 to 1.5% or at least 1.5 - 2.0%.

Instructions on conventional genetic engineering methods, such as, for example, isolation of chromosomal DNA, plasmid DNA, handling of restriction enzymes etc., are found in Sambrook et al. (Molecular Cloning - A Laboratory Manual (1989) Cold Spring Harbor Laboratory Press). Instructions on transformation and conjugation in coryneform bacteria are found, inter alia, in Thierbach et al. (Applied Microbiology and Biotechnology 29, 356-362 (1988)), in Schäfer et al. (Journal of Bacteriology 172, 1663-1666 (1990) and Gene 145, 69-73 (1994)) and in Schwarzer and Pühler (Bio/Technology 9, 84-87. (1991)).

Vectors which replicate to only a limited extent are understood as meaning plasmid vectors which, as a function of the conditions under which the host or carrier is cultured, replicate or do not replicate. Thus, a temperature-sensitive plasmid for coryneform bacteria which can replicate only at temperatures below 31ºC has been described by Nakamura et al. (US-A-6,303,383).

The description also provides coryneform bacteria, in particular of the genus Corynebacterium, which produce L-lysine, characterized in that
a) instead of the singular copy of an open reading frame (ORF), a gene or allele of lysine production naturally present at the particular desired site (locus), these have at least two copies of the said open reading frame (ORF), gene or allele, preferably in tandem arrangement, no nucleotide sequence which is capable of/enables episomal replication in microorganisms, no nucleotide sequence which is capable of/enables transposition and no nucleotide sequence which imparts resistance to antibiotics being present at the particular site, and in that these
b) optionally have at least a third copy of the said open reading frame (ORF), gene or allele of L-lysine production at a further gene site, no nucleotide sequence which is capable of/enables episomal replication in microorganisms, no nucleotide sequence which is capable of/enables transposition and no nucleotide sequence which imparts resistance to antibiotics being present at the further gene site.

The description also furthermore provides a process for the preparation of L-lysine, which comprises the following steps:
a) fermentation of coryneform bacteria, in particular of the genus Corynebacterium, which
   i) instead of the singular copy of an open reading frame (ORF), gene or allele of lysine production present at the particular desired site (locus), have at least two copies of the open reading frame (ORF), gene or allele in question, preferably in tandem arrangement, no nucleotide sequence which is capable of/enables episomal replication in microorganisms, no nucleotide sequence which is capable of/enables transposition and no nucleotide sequence which imparts resistance to antibiotics being present at the particular site, and in that these
   ii) optionally have at least a third copy of the open reading frame (ORF), gene or allele of L-lysine production in question at a further gene site, no nucleotide sequence which is capable of/enables episomal replication in microorganisms, no nucleotide sequence which is capable of/enables transposition and no nucleotide sequence which imparts resistance to antibiotics being present at the further gene site,
      under conditions which allow expression of the said open reading frames (ORFs), genes or alleles,
b) concentration of the L-lysine in the fermentation broth,
c) isolation of the L-lysine from the fermentation broth, optionally
d) with constituents from the fermentation broth and/or the biomass to the extent of > (greater than) 0 to 100%.

A "copy of an open reading frame (ORF), gene or allele of lysine production" is to be understood as meaning all the, preferably endogenous, open reading frames, genes or alleles of which enhancement/over-expression can have the effect of improving lysine production. Enhancement is understood as meaning an increase in the intracellular concentration or activity of the particular gene product, protein or enzyme.

These include, inter alia, the following genes: accBC, accDA, cstA, cysD, cysE, cysH, cysK, cysN, cysQ, dapA, dapB, dapC, dapD, dapE, dapF, ddh, dps, eno, gap, gap2, gdh, gnd, lysC, lysC^{FBR}, lysE, msiK, opcA, oxyR, ppc, ppc^{FBR}, pgk, pknA, pknB, pknD, pknG, ppsA, ptsH, ptsI, ptsM, pyc, pyc P458S, sigC, sigD, sigE, sigH, sigM, tal, thyA, tkt, tpi, zwa1, zwf and zwf A213T. These are summarized and explained in Table 1.

These include, in particular, the lysC^{FBR} alleles which code for a "feed back" resistant aspartate kinase. Various lysC^{FBR} alleles are summarized and are explained in Table 2.

The following lysC^{FBR} alleles are preferred: lysC A279T (replacement of alanine at position 279 of the aspartate kinase protein coded, according to SEQ ID NO: 2, by threonine), lysC A279V (replacement of alanine at position 279 of the aspartate kinase protein coded, according to SEQ ID NO: 2, by valine), lysC S301F (replacement of serine at position 301 of the aspartate kinase protein coded, according to SEQ ID NO: 2, by phenylalanine), lysC T308I (replacement of threonine at position 308 of the aspartate kinase protein coded, according to SEQ ID NO: 2, by isoleucine), lysC S301Y (replacement of serine at position 308 of the aspartate kinase protein coded, according to SEQ ID NO: 2, by tyrosine), lysC G345D (replacement of glycine at position 345 of the aspartate kinase protein coded, according to SEQ ID NO: 2, by aspartic acid), lysC R320G (replacement of arginine at position 320 of the aspartate kinase protein coded, according to SEQ ID NO: 2, by glycine), lysC T311I (replacement of threonine at position 311 of the aspartate kinase protein coded, according to SEQ ID NO: 2, by isoleucine), lysC S381F (replacement of serine at position 381 of the aspartate kinase protein coded, according to SEQ ID NO: 2, by phenylalanine).

The lysC^{FBR} allele lysC T311I (replacement of threonine at position 311 of the aspartate kinase protein coded, according to SEQ ID NO: 2, by isoleucine), the nucleotide sequence of which is shown as SEQ ID NO:3, is particularly preferred; the amino acid sequence of the aspartate kinase protein coded is shown as SEQ ID NO:4.

The following open reading frames, genes or nucleotide sequences, inter alia, can be used as the "further gene site" which is not essential for growth or lysine production: aecD, ccpA1, ccpA2, citA, citB, citE, fda, gluA, gluB, gluC, gluD, luxR, luxS, lysR1, lysR2, lysR3, menE, mqo, pck, pgi, poxB and zwa2, in particular the genes aecD, gluA, gluB, gluC, gluD and pck. These are summarized and explained in Table 3. Intergenic regions in the chromosome, that is to say nucleotide sequences without a coding function, can furthermore be used. Finally, prophages or defective phages contained in the chromosome can be used.

**Table 1**

| Open reading frames, genes and alleles of lysine production | | | |
|---|---|---|---|
| Name | Description of the coded enzyme or protein | Reference | Access Number |
| accBC | Acyl-CoA Carboxylase EC 6.3.4.14 (acyl-CoA carboxylase) | Jäger et al. Archives of Microbiology (1996) 166:76-82 | U35023 |
| | | EP1108790; | AX123524 |
| | | WO0100805 | AX066441 |
| accDA | Acetyl-CoA Carboxylase EC 6.4.1.2 (acetyl-CoA carboxylase) | EP1055725 | |
| | | EP1108790 | AX121013 |
| | | WO0100805 | AX066443 |
| cstA | Carbon Starvation Protein A (carbon starvation protein A) | EP1108790 | AX120811 |
| | | WO0100804 | AX066109 |
| cysD | Sulfate Adenylyltransferase sub-unit II EC 2.7.7.4 (sulfate adenylyltransferase small chain) | EP1108790 | AX123177 |
| cysE | Serine Acetyltransferase EC 2.3.1.30 (serine acetyltransferase) | EP1108790 | AX122902 |
| | | WO0100843 | AX063961 |
| cysH | 3'-Phosphoadenyl Sulfate Reductase EC 1.8.99.4 (3'-phosphoadenosine 5'-phosphosulfate reductase) | EP1108790 | A123178 |
| | | WO0100842 | AX066001 |
| cysK | Cysteine Synthase EC 4.2.99.8 (cysteine synthase) | EP1108790 | AX122901 |
| | | WO0100843 | AX063963 |
| cysN | Sulfate Adenylyltransferase sub-unit I EC 2,7.7.4 (sulfate adenylyltransferase) | EP1108790 | AX123176 AX127152 |
| cysQ | Transport protein CysQ (transporter cysQ) | EP1108790 | AX127145 |
| | | WO0100805 | AX066423 |
| dapA | Dihydrodipicolinate Synthase EC 4.2.1.52 (dihydrodipicolinate synthase) | Bonnassie et al. Nucleic Acids Research 18:6421 (1990) | X53993 |
| | | Pisabarro et al., Journal of Bacteriology 175:2743-2749(1993) | Z21502 |
| | | EP1108790 | |
| | | WO0100805 | |
| | | EP0435132 | |
| | | EP1067192 | AX123560 |
| | | EP1067193 | AX063773 |
| dapB | Dihydrodipicolinate Reductase EC 1.3.1.26 (dihydrodipicolinate reductase) | EP1108790 | AX127149 |
| | | WO0100843 | AX063753 |
| | | EP1067192 | AX137723 |
| | | EP1067193 | AX137602 |
| | | Pisabarro et al., Journal of Bacteriology 175:2743- 2749(1993) | X67737 |
| | | | Z21502 |
| | | JP1998215883 | E16749 |
| | | JP1997322774 | E14520 |
| | | JP1997070291 | E12773 |
| | | JP1995075578 | E08900 |
| dapC | N-Succinyl Aminoketopimelate Transaminase EC 2.6.1.17 (N-succinyl diaminopimelate transaminase) | EP1108790 | AX127146 |
| | | WO010084 | AX064219 |
| | | EP1136559 | |
| dapD | Tetrahydrodipicolinate Succinylase EC 2.3.1.117 (tetrahydrodipicolinate succinylase) | EP1108790 | AX127146 |
| | | WO010084 | AX063757 |
| | | Wehrmann et al. Journal of Bacteriology 180:3159-3165(1998) | AJ004934 |
| dapE | N-Succinyl Diaminopimelate Desuccinylase EC 3.5.1.18 (N-succinyl diaminopimelate desuccinylase) | EP1108790 | AX127146 |
| | | WO0100843 | AX063749 |
| | | Wehrmann et al. Microbiology 140:3349-3356 (1994) | X81379 |
| dapF | Diaminopimelate Epimerase EC 5.1.1.7 (diaminopimelate epimerase) | EP1108790 | AX127149 |
| | | WO0100843 | AX063719 |
| | | EP1085094 | AX137620 |
| ddh | Diaminopimelate Dehydrogenase EC 1.4.1.16 (diaminopimelate dehydrogenase) | EP1108790 | AX127152 |
| | | WO0100843 | AX063759 |
| | | Ishino et al., Nucleic Acids Research 15:3917-3917(1987) | Y00151 |
| | | JP1997322774 | E14511 |
| | | JP1993284970 | E05776 |
| | | Kim et al., Journal of Microbiology and Biotechnology 5:250-256(1995) | D87976 |
| dps | DNA Protection Protein (protection during starvation protein) | EP1108790 | AX127153 |
| eno | Enolase EC 4.2.1.11 (enolase) | EP1108790 | AX127146 |
| | | WO0100844 | AX064945 |
| | | EP1090998 | AX136862 |
| | | Hermann et al., Electrophoresis 19:3217-3221 (1998) | |
| gap | Glyceraldehyde 3-Phosphate Dehydrogenase EC 1.2.1.12 | EP1108790 | AX127148 |
| | | WO010084 | AX064941 |
| | (glyceraldehyde 3-phosphate dehydrogenase) | Eikmanns et al., Journal of Bacteriology 174:6076-6086(1992) | X59403 |
| gap2 | Glyceraldehyde 3-Phosphate Dehydrogenase EC 1.2.1.12 (glyceraldehyde 3-phosphate dehydrogenase 2) | EP1108790 | AX127146 |
| | | WO0100844 | AX064939 |
| gdh | Glutamate Dehydrogenase EC 1.4.1.4 (glutamate dehydrogenase) | EP1108790 | AX127150 |
| | | WO0100844 | AX063811 |
| | | Boermann et al., Molecular Microbiology 6:317-326 (1992). | X59404 |
| | | Guyonvarch et al. NCBI | X72855 |
| gnd | 6-Phosphogluconate Dehydrogenase EC 1.1.1.44 (6-phosphogluconate dehydrogenase) | EP1108790 | AX127147 |
| | | | AX121689 |
| | | WO0100844 | AX065125 |
| lysC | Aspartate Kinase EC 2.7.2.4 (aspartate kinase) | EP1108790 | AX120365 |
| | | WO010084 | AX063743 |
| | | Kalinowski et al., Molecular Microbiology 5:1197-204 (1991) | X57226 |
| lysC^{FB} R | Aspartate Kinase feedback resistent (fbr) EC 2.7.2.4 (aspartate kinase fbr) | see Table 2 | |
| lysE | Lysine Exporter (lysine exporter protein) | EP1108790 | AX123539 |
| | | WO010084 | AX123539 |
| | | Vrljic et al., Molecular Microbiology 22:815-826 (1996) | X96471 |
| msiK | Sugar Importer (multiple sugar import protein) | EP1108790 | AX120892 |
| opcA | Glucose 6-Phosphate Dehydrogenase (subunit of glucose 6-phosphate dehydrogenase) | WO0104325 | AX076272 |
| oxyR | Transcription Regulator (transcriptional regulator) | EP1108790 | AX122198 |
| | | | AX127149 |
| ppc^{FBR} | Phosphoenol Pyruvate Carboxylase feedback resistent EC 4.1.1.31 (phosphoenol pyruvate carboxylase feedback resistant) | EP0723011 WO0100852 | |
| ppc | Phosphoenol Pyruvate Carboxylase EC 4.1.1.31 (phosphoenol pyruvate carboxylase) | EP1108790 | AX127148 |
| | | | AX123554 |
| | | O'Reagan et al., Gene 77 (2) :237-251(1989) | M25819 |
| pgk | Phosphoglycerate Kinase | EP1108790 | AX121838 |
| | EC 2.7.2.3 (phosphoglycerate kinase) | | AX127148 |
| | | WO0100844 | AX064943 |
| | | Eiknanns, Journal of Bacteriology 174:6076-6086 (1992) | X59403 |
| pknA | Protein Kinase A (protein kinase A) | EP1108790 | AX120131 |
| | | | AX120085 |
| pknB | Protein Kinase B (protein kinase B) | EP1108790 | AX120130 |
| | | | AX120085 |
| pknD, | Protein Kinase D (protein kinase D) | EP1108790 | AX127150 |
| | | | AX122469 |
| | | | AX122468 |
| pknG | Protein Kinase G (protein kinase G) | EP1108790 | AX127152 |
| | | | AX123109 |
| ppsA | Phosphoenol Pyruvate Synthase EC 2.7.9.2 (phosphoenol pyruvate synthase) | EP1108790 | AX127144 |
| | | | AX120700 |
| | | | AX122469 |
| ptsH | Phosphotransferase System Protein H EC 2.7.1.69 (phosphotransferase system component H) | EP1108790 | AX122210 |
| | | | AX127149 |
| | | WO0100844 | AX069154 |
| ptsI | Phosphotransferase System Enzyme I EC 2.7.3.9 (phosphotransferase system enzyme I) | EP1108790 | AX122206 |
| | | | AX127149 |
| ptsM | Glucose-specific Phosphotransferase System Enzyme II EC 2.7.1.69 (glucose phosphotransferase-system enzyme II) | Lee et al., FEMS Microbiology Letters 119(1-2):137-145 (1994) | L18874 |
| pyc | Pyruvate Carboxylase EC 6.4.1.1 (pyruvate carboxylase) | WO9918228 | A97276 |
| | | Peters-Wendisch et al., Microbiology 144:915-927 (1998) | Y09548 |
| pyc P458S | Pyruvate Carboxylase EC 6.4.1.1 (pyruvate carboxylase) amino acid exchange P458S | EP1108790 | |
| sigC | Sigma Factor C EC 2.7.7.6 (extracytoplasmic function alternative sigma factor C) | EP1108790 | AX120368 |
| | | | AX120085 |
| sigD | RNA Polymerase Sigma Factor D EC 2.7.7.6 (RNA polymerase sigma factor) | EP1108790 | AX120753 |
| | | | AX127144 |
| sigE | Sigma Factor E EC 2.7.7.6 (extracytoplasmic function alternative sigma factor E) | EP1108790 | AX127146 |
| | | | AX121325 |
| sigH | Sigma Factor H EC 2.7.7.6 (sigma factor SigH) | EP1108790 | AX127145 |
| | | | AX120939 |
| sigM | Sigma Factor M EC 2.7.7.6 (sigma factor SigM) | EP1108790 | AX123500 |
| | | | AX127153 |
| tal | Transaldolase EC 2.2.1.2 (transaldolase) | WO0104325 | AX076272 |
| thyA | Thymidylate Synthase EC 2.1.1.45 (thymidylate synthase) | EP1108790 | AX121026 |
| | | | AX127145 |
| tkt | Transketolase EC 2.2.1.1 (transketolase) | Ikeda et al., NCBI | AB023377 |
| tpi | Triose Phosphate Isomerase EC 5.3.1.1 (triose phosphate isomerase) | Eikmanns, Journal of Bacteriology 174:6076-6086 (1992) | X59403 |
| zwa1 | Cell Growth Factor 1 (growth factor 1) | EP1111062 | AX133781 |
| zwf | Glucose 6-Phosphate 1-Dehydrogenase EC 1.1.1.49 (glucose 6-phosphate 1-dehydrogenase) | EP1108790 | AX127148 |
| | | | AX121827 |
| | | WO0104325 | AX076272 |
| zwf A213T | Glucose 6-Phosphate 1-Dehydrogenase EC 1.1.1.49 (glucose 6-phosphate 1-dehydrogenase) amino acid exchange A213T | EP1108790 | |

**Table 2**

| lysC^{FBR} alleles which code for feed back resistant aspartate kinases | | | |
|---|---|---|---|
| Name of the allele | Amino acid replacement | Reference | Access Number |
| lysC^{FM}-E05108 | | JP 1993184366-A (sequence 1) | E05108 |
| lysC^{FBR}-E06825 | lysC A279T | JP 1994062866-A (sequence 1) | E06825 |
| lysC^{FBR}-E06826 | lysC A279T | JP 1994062866-A (sequence 2) | E06826 |
| lysC^{FBR}-E06827 | | JP 1994062866-A (sequence 3) | E06827 |
| lysC^{FBR}-E08177 | | JP 1994261766-A (sequence 1) | E08177 |
| lysC^{FBR}-E08178 | lysC A279T | JP 1994261766-A (sequence 2) | E08178 |
| lysC^{FBR}-E08179 | lysC A279V | JP 1994261766-A (sequence 3) | E08179 |
| lysC^{FBR}-E08180 | lysC S301F | JP 1994261766-A (sequence 4) | E08180 |
| lysC^{FBR}-E08181 | lysC T308I | JP 1994261766-A (sequence 5) | E08181 |
| lysC^{FBR}-E08182 | | JP 1994261766-A | E08182 |
| lysC^{FBR}-E12770 | | JP 1997070291-A (sequence 13) | E12770 |
| lysC^{FBR}-E14514 | | JP 1997322774-A (sequence 9) | E14514 |
| lysC^{FBR}-E163 52 | | JP 1998165180-A (sequence 3) | E16352 |
| lysC^{FBR}-E16745 | | JP 1998215883-A (sequence 3) | E16745 |
| lysC^{FBR}-E16746 | | JP 1998215883-A (sequence 4) | E16746 |
| lySC^{FBR}- 174588 | | US 5688671-A (sequence 1) | I74588 |
| lysC^{FBR}-I74589 | lysC A279T | US 5688671-A (sequence 2) | I74589 |
| lysC^{FBR}-I74590 | | US 5688671-A (sequence 7) | I74590 |
| lysC^{FBR}-I74591 | lysC A279T | US 5688671-A (sequence 8) | I74591 |
| lysC^{FBR}-I74592 | | US 5688671-A (sequence 9) | I74592 |
| lysC^{FBR}-I74593 | lysC A279T | US 5688671-A (sequence 10) | I74593 |
| lysC^{FBR}-I74594 | | US 5688671-A (sequence 11) | I74594 |
| lysC^{FBR}-I74595 | lysC A279T | US 5688671-A (sequence 12) | I74595 |
| lysC^{FBR}-I74596 | | US 5688671-A (sequence 13) | I74596 |
| lysC^{FBR}-I74597 | lysC A279T | US 5688671-A (sequence 14) | I74597 |
| lysC^{FBR}-X57226 | lysC S301Y | EP0387527 Kalinowski et al., Molecular and General Genetics 224:317-324 (1990) | X57226 |
| lysC^{FBR}-L16848 | lysC G345D | Follettie and Sinskey NCBI Nucleotide Database (1990) | L16848 |
| lysC^{FBR}-L27125 | lysC R320G lysC G345D | Jetten et al., Applied Microbiology Biotechnology 43:76-82 (1995) | L27125 |
| lysC^{FBR} | lysC T311I | WO0063388 (sequence 17) | |
| lysC^{FBR} | lysC S301F | US3732144 | |
| lysC^{FBR} | lysC S381F | | |
| lysC^{FBR} | | JP6261766 (sequence 1) | |
| lysC^{FBR} | lysC A279T | JP6261766 (sequence 2) | |
| lysC^{FBR} | lysC A279V | JP6261766 (sequence 3) | |
| lysC^{FBR} | lysC S301F | JP6261766 (sequence 4) | |
| lysC^{FBR} | lysC T308I | JP6261766 (sequence 5) | |

**Table 3**

| Further gene sites for integration of open reading frames, genes and alleles of lysine production | | | |
|---|---|---|---|
| Gene name | Description of the coded enzyme or protein | Reference | Access Number |
| aecD | beta C-S Lyase EC 2.6.1.1 (beta C-S lyase) | Rossol et al., Journal of Bacteriology 174(9):2968-77 (1992) | M89931 |
| ccpA1 | Catabolite Control Protein (catabolite control protein A1) | W00100844 | AX065267 |
| | | EP1108790 | AX127147 |
| ccpA2 | Catabolite Control Protein (catabolite control protein A2) | WO0100844 | AX065267 |
| | | EP1108790 | AX121594 |
| citA | Sensor Kinase CitA (sensor kinase CitA) | EP1108790 | AX120161 |
| citB | Transcription Regulator CitB (transcription regulator CitB) | EP1108790 | AX120163 |
| citE | Citrate Lyase EC 4.1.3.6 (citrate lyase) | WO010084 | AX065421 |
| | | EP1108790 | AX127146 |
| fda | Fructose Bisphosphate Aldolase EC 4.1.2.13 (fructose 1,6-bisphosphate aldolase) | von der Osten et al., Molecular Microbiology 3(11):1625-37 (1989) | X17313 |
| gluA | Glutamate Transport ATP-binding Protein (glutamate transport ATP-binding protein) | Kronemeyer et al., Journal of Bacteriology 177(5):1152-8 (1995) | X81191 |
| gluB | Glutamate-binding Protein (glutamate-binding | Kronemeyer et al., Journal of Bacteriology 177(5):1152-8 (1995) | X81191 |
| | protein) | | |
| gluC | Glutamate Transport Permease (glutamate transport system permease) | Kronemeyer et al., Journal of Bacteriology 177(5):1152-8 (1995) | X81191 |
| gluD | Glutamate Transport Permease (glutamate transport system permease) | Kronemeyer et al., Journal of Bacteriology 177(5):1152-8 (1995) | X81191 |
| luxR | Transcription Regulator | WO010084 | AX065953 |
| | LuxR (transcription regulator LuxR) | EP1108790 | AX123320 |
| luxS | Histidine Kinase LuxS | EP1108790 | AX123323 |
| | (histidine kinase LuxS) | | AX127153 |
| lysR1 | Transcription Regulator | EP1108790 | AX064673 |
| | LysR1 | | AX127144 |
| | (transcription regulator LysR1 | | |
| lysR2 | Transcription Activator | EP1108790 | AX123312 |
| | LysR2 | | |
| | (transcription regulator LysR2) | | |
| lysR3 | Transcription Regulator | WO010084 | AX065957 |
| | LysR3 | EP1108790 | AX127150 |
| | (transcription regulator LysR3) | | |
| menE | O-Succinylbenzoic Acid | WO010084 | AX064599 |
| | CoA Ligase | EP1108790 | AX064193 |
| | EC 6.2.1.26 (O-succinylbenzoate CoA ligase) | | AX127144 |
| mqo | Malate-Quinone Oxidoreductase (malate-quinone-oxidoreductase) | Molenaar et al., Eur. Journal of Biochemistry 1;254(2):395-403 (1998) | AJ224946 |
| pck | Phosphoenol Pyruvate | WO010084 | AJ269506 |
| | Carboxykinase (phosphoenol pyruvate | | AX065053 |
| | carboxykinase) | | |
| pgi | Glucose 6-Phosphate | EP1087015 | AX136015 |
| | Isomerase EC 5.3.1.9 (glucose-6-phosphate isomerase) | EP1108790 | A127146 |
| poxB | Pyruvate Oxidase | WO010084 | AX064959 |
| | EC 1.2.3.3 (pyruvate oxidase) | EP1096013 | AX137665 |
| zwa2 | Cell Growth Factor 2 | EP1106693 | AX113822 |
| | (growth factor 2) | EP1108790 | AX127146 |

The description accordingly also provides a process for the production of coryneform bacteria which produce L-lysine, characterized in that
a) the nucleotide sequence of a desired ORF, gene or allele of lysine production, optionally including the expression and/or regulation signals, is isolated
b) at least two copies of the nucleotide sequence of the ORF, gene or allele of lysine production are arranged in a row, preferably in tandem arrangement
c) the nucleotide sequence obtained according to b) is incorporated in a vector which does not replicate or replicates to only a limited extent in coryneform bacteria,
d) the nucleotide sequence according to b) or c) is transferred into coryneform bacteria, and
e) coryneform bacteria which have at least two copies of the desired ORF, gene or allele of lysine production at the particular desired natural site instead of the singular copy of the ORF, gene or allele originally present are isolated, no nucleotide sequence which is capable of/enables episomal replication in microorganisms, no nucleotide sequence which is capable of/enables transposition and no nucleotide sequence which imparts resistance to antibiotics remaining at the particular natural site (locus), and optionally
f) at least a third copy of the open reading frame (ORF), gene or allele of lysine production in question is introduced at a further gene site, no nucleotide sequence which is capable of/enables episomal replication in microorganisms, no nucleotide sequence which is capable of/enables transposition and no nucleotide sequence which imparts resistance to antibiotics remaining at the further gene site.

The description also provides coryneform bacteria, in particular of the genus Corynebacterium, which produce L-methionine and/or L-threonine, characterized in that
a) instead of the singular copy of an open reading frame (ORF), a gene or allele of methionine production or threonine production naturally present at the particular desired site (locus), these have at least two copies of the said open reading frame (ORF), gene or allele, preferably in tandem arrangement, no nucleotide sequence which is capable of/enables episomal replication in microorganisms, no nucleotide sequence which is capable of/enables transposition and no nucleotide sequence which imparts resistance to antibiotics being present at the particular site, and in that these
b) optionally have at least a third copy of the open reading frame (ORF), gene or allele of methionine production or threonine production mentioned at a further gene site, no nucleotide sequence which is capable of/enables episomal replication in microorganisms, no nucleotide sequence which is capable of/enables transposition and no nucleotide sequence which imparts resistance to antibiotics being present at the further gene site.

The description also furthermore provides a process for the preparation of L-methionine and/or L-threonine, which comprises the following steps:
a) fermentation of coryneform bacteria, in particular of the genus Corynebacterium, which
   i) instead of the singular copy of an open reading frame (ORF), gene or allele of methionine production or threonine production present at the particular desired site (locus), have at least two copies of the open reading frame (ORF), gene or allele in question, preferably in tandem arrangement, no nucleotide sequence which is capable of/enables episomal replication in microorganisms, no nucleotide sequence which is capable of/enables transposition and no nucleotide sequence which imparts resistance to antibiotics being present at the particular site, and
   ii) optionally have at least a third copy of the open reading frame (ORF), gene or allele of methionine production or threonine production in question at a further gene site, no nucleotide sequence which is capable of/enables episomal replication in microorganisms, no nucleotide sequence which is capable of/enables transposition and no nucleotide sequence which imparts resistance to antibiotics being present at the further gene site,
      under conditions which allow expression of the said open reading frames (ORFs), genes or alleles,
b) concentration of the L-methionine and/or L-threonine in the fermentation broth,
c) isolation of the L-methionine and/or L-threonine from the fermentation broth, optionally
d) with constituents from the fermentation broth and/or the biomass to the extent of > (greater than) 0 to 100%.

A "copy of an open reading frame (ORF), gene or allele of methionine production" is to be understood as meaning all the, preferably endogenous, open reading frames, genes or alleles of which enhancement/over-expression can have the effect of improving methionine production.

These include, inter alia, the following genes: accBC, accDA, aecD, cstA, cysD, cysE, cysH, cysK, cysN, cysQ, dps, eno, fda, gap, gap2, gdh, gnd, glyA, hom, hom^{FBR}, lysC, lysC^{FBR}, metA, metB, metE, metH, metY, msiK, opcA, oxyR, ppc, ppc^{FBR}, pgk, pknA, pknB, pknD, pknG, ppsA, ptsH, ptsI, ptsM, pyc, pyc P458S, sigC, sigD, sigE, sigH, sigM, tal, thyA, tkt, tpi, zwa1 zwf and zwf A213T. These are summarized and explained in Table 4. These include, in particular, the lysC^{FBR}alleles which code for a "feed back" resistant aspartate kinase (see Table 2) and the hom^{FBR} alleles which code for a "feed back" resistant homoserine dehydrogenase.

The at least third, optionally fourth or fifth copy of the open reading frame (ORF), gene or allele of methionine production in question can be integrated at a further site. The following genes, inter alia, can be used for this: brnE, brnF, brnQ, ccpA1, ccpA2, citA, citB, citE, ddh, gluA, gluB, gluC, gluD, luxR, luxS, lysR1, lysR2, lysR3, menE, metD, metK, pck, pgi, poxB and zwa2. These are summarized and explained in Table 5. Intergenic regions in the chromosome, that is to say nucleotide sequences without a coding function, can furthermore be used. Finally, prophages or defective phages contained in the chromosome can be used for this.

**Table 4**

| Open reading frames, genes and alleles of methionine production | | | |
|---|---|---|---|
| Name | Description of the coded enzyme or protein | Reference | Access Number |
| accBC | Acyl-CoA Carboxylase EC 6.3.4.14 (acyl-CoA carboxylase) | Jager et al. Archives of Microbiology (1996) 166:76-82 | U35023 |
| | | EP1108790; | AX123524 |
| | | WO0100805 | AX066441 |
| accDA | Acetyl-CoA Carboxylase | EP1055725 | |
| | EC 6.4.1.2 | EP1108790 | AX121013 |
| | (acetyl-CoA carboxylase) | WO0100805 | AX066443 |
| aecD | Cystathionine beta-Lyase EC 4.4.1.8 (cystathionine beta-lyase) | Rossol et al., Journal of Bacteriology 174:2968-2977 (1992) | M89931 |
| cstA | Carbon Starvation Protein A | EP1108790 | AX120811 |
| | (carbon starvation protein A) | WO01008 | AX066109 |
| cysD | Sulfate Adenylyltransferase sub-unit II EC 2.7.7.4 (sulfate adenylyltransferase small chain) | EP1108790 | AX123177 |
| cysE | Serine Acetyltransferase | EP1108790 | AX122902 |
| | EC 2.3.1.30 (serine acetyltransferase) | WO0100843 | AX063961 |
| cysH | 3'-Phosphoadenyl Sulfate Reductase | EP1108790 | AX123178 |
| | EC 1.8.99.4 (3'-phosphoadenosine 5'-phosphosulfate reductase) | WO010084 | AX066001 |
| cysK | Cysteine Synthase | EP1108790 | AX122901 |
| | EC 4.2.99.8 (cysteine synthase) | WO0100843 | AX063963 |
| cysN | Sulfate Adenylyltransferase sub- | EP1108790 | A123176 |
| | unit I EC 2.7.7.4 (sulfate adenylyltransferase) | | AX127152 |
| cysQ | Transport protein CysQ | EP1108790 | AX127145 |
| | (transporter cysQ) | WO0100805 | AX066423 |
| dps | DNA Protection Protein (protection during starvation protein) | EP1108790 | AX127153 |
| eno | Enolase | EP1108790 | AX127146 |
| | EC 4.2.1.11 | WO0100844 | AX064945 |
| | (enolase) | EP1090998 | AX136862 |
| | | Hermann et al., Electrophoresis 19:3217-3221 (1998) | |
| fda | Fructose Bisphosphate Aldolase EC 4.1.12.13 | van der Osten et al., Molecular | X17313 |
| | (fructose bisphosphate aldolase) | Microbiology 3:1625-1637 (1989) | |
| gap | Glyceraldehyde 3-Phosphate | EP1108790 | AX127148 |
| | Dehydrogenase | WO0100844 | AX064941 |
| | EC 1.2.1.12 (glyceraldehyde 3-phosphate dehydrogenase) | Eikmanns et al., Journal of Bacteriology 174:6076-6086(1992) | X59403 |
| gap2 | Glyceraldehyde 3-Phosphate | EP1108790 | AX127146 |
| | Dehydrogenase | WO0100844 | AX064939 |
| | EC 1.2.1.12 (glyceraldehyde 3-phosphate dehydrogenase 2) | | |
| gdh | Glutamate Dehydrogenase | EP1108790 | AX127150 |
| | EC 1.4.1.4 | WO0100844 | AX063811 |
| | (glutamate dehydrogenase) | Boermann et al., Molecular Microbiology 6:317-326 (1992) | X59404 |
| | | Guyonvarch et al, NCBI | X72855 |
| glyA | Glycine/Serine | EP1108790 | AX127146 |
| | Hydroxymethyltransferase EC 2.1.2.1 (glycine/serine hydroxymethyltransferase) | | AX121194 |
| gnd | 6-Phosphogluconate Dehydrogenase | EP1108790 | AX127147 |
| | EC 1.1.1.44 | | AX121689 |
| | (6-phosphogluconate dehydrogenase) | WO0100844 | AX065125 |
| hom | Homoserine Dehydrogenase EC 1.1.1.3 (homoserine dehydrogenase) | Peoples et al., Molecular Microbiology 2:63-72 (1988) | Y00546 |
| hom^{FBR} | Homoserine Dehydrogenase feedback resistant (fbr) EC 1.1.1.3 (homoserine dehydrogenase fbr) | Reinscheid et al., Journal of Bacteriology 173:3228-30 (1991) | |
| lysC | Aspartate Kinase | EP1108790 | AX120365 |
| | EC 2.7.2.4 | WO0100844 | AX063743 |
| | (aspartate kinase) | Kalinowski et al., Molecular Microbiology 5:1197-204 (1991) | X57226 |
| lysC^{FB} R | Aspartate Kinase feedback resistent (fbr) EC 2.7.2.4 (aspartate kinase fbr) | see Table 2 | |
| metA | Homoserine Acetyltransferase EC 2.3.1.31 (homoserine acetyltransferase) | Park et al., Molecular Cells 8:286-94 (1998) | AF052652 |
| metB | Cystathionine γ-Lyase EC 4.4.1.1 (cystathionine gamma-synthase) | Hwang et al., Molecular Cells 9:300-308 (1999) | AF126953 |
| metE | Homocysteine Methyltransferase | EP1108790 | AX127146 |
| | EC 2.1.1.14 | | AX121345 |
| | (homocysteine methyltransferase) | | |
| metH | Homocysteine Methyltransferase | EP1108790 | AX127148 |
| | (Vitamin B12-dependent) EC 2.1.1.14 (homocysteine methyltransferase) | | AX121747 |
| metY | Acetylhomoserine Sulfhydrolase | EP1108790 | AX120810 |
| | (acetylhomoserine sulfhydrolase) | | AX127145 |
| msiK | Sugar Importer (multiple sugar import protein) | EP1108790 | AX120892 |
| opcA | Glucose 6-Phosphate Dehydrogenase (subunit of glucose 6-phosphate dehydrogenase) | WO0104325 | AX076272 |
| oxyR | Transcription Regulator | EP1108790 | AX122198 |
| | (transcriptional regulator) | | AX127149 |
| ppc^{FBR} | Phosphoenol Pyruvate Carboxylase | EP0723011 | |
| | feedback resistent EC 4.1.1.31 | W00100852 | |
| | (phosphoenol pyruvate carboxylase feedback resistant) | | |
| ppc | Phosphoenol Pyruvate Carboxylase | EP1108790 | AX127148 |
| | EC 4.1.1.31 | | AX123554 |
| | (phosphoenol pyruvate carboxylase) | O'Reagan et al., Gene 77(2):237-251(1989) | M25819 |
| pgk | Phosphoglycerate Kinase | EP1108790 | AX121838 |
| | EC 2.7.2.3 | | AX127148 |
| | (phosphoglycerate kinase) | WO0100844 | AX064943 |
| | | Eikmanns, Journal of Bacteriology 174:6076-6086 (1992) | X59403 |
| pknA | Protein Kinase A | EP1108790 | AX120131 |
| | (protein kinase A) | | AX120085 |
| pknB | Protein Kinase B | EP1108790 | AX120130 |
| | (protein kinase B) | | AX120085 |
| pknD | Protein Kinase D | EP1108790 | AX127150 |
| | (protein kinase D) | | AX122469 |
| | | | AX122468 |
| pknG | Protein Kinase G | EP1108790 | AX127152 |
| | (protein kinase G) | | AX123109 |
| ppsA | Phosphoenol Pyruvate Synthase | EP1108790 | AX127144 |
| | EC 2.7.9.2 | | AX120700 |
| | (phosphoenol pyruvate synthase) | | AX122469 |
| ptsH | Phosphotransferase System Protein | EP1108790 | AX122210 |
| | H | | AX127149 |
| | EC 2.7.1.69 (phosphotransferase system component H) | WO0100844 | AX069154 |
| ptsI | Phosphotransferase System Enzyme I | EP1108790 | A122206 |
| | EC 2.7.3.9 (phosphotransferase system enzyme I) | | AX127149 |
| ptsM | Glucose-specific Phosphotransferase System Enzyme II EC 2.7.1.69 (glucose phosphotransferase-system enzyme II) | Lee et al., FEMS Microbiology Letters 119(1-2):137-145 (1994) | L18874 |
| pyc | Pyruvate Carboxylase | WO9918228 | A97276 |
| | EC 6.4.1.1 (pyruvate carboxylase) | Peters-Wendisch et al., Microbiology 144:915-927 (1998) | Y09548 |
| pyc P458S | Pyruvate Carboxylase EC 6.4.1.1 (pyruvate carboxylase) amino acid exchange P458S | EP1108790 | |
| sigC | Sigma Factor C | EP1108790 | AX120368 |
| | EC 2.7.7.6 | | AX120085 |
| | (extracytoplasmic function alternative sigma factor C) | | |
| sigD | RNA Polymerase Sigma Factor D | EP1108790 | AX120753 |
| | EC 2.7.7.6 | | AX127144 |
| | (RNA polymerase sigma factor) | | |
| sigE | Sigma Factor E | EP1108790 | AX127146 |
| | EC 2.7.7.6 | | AX121325 |
| | (extracytoplasmic function alternative sigma factor E) | | |
| sigH | Sigma Factor H | EP1108790 | AX127145 |
| | EC 2.7.7.6 | | AX120939 |
| | (sigma factor SigH) | | |
| sigM | Sigma Factor M | EP1108790 | AX123500 |
| | EC 2.7.7.6 | | AX127153 |
| | (sigma factor SigM) | | |
| tal | Transaldolase | WO0104325 | AX076272 |
| | EC 2.2.1.2 | | |
| | (transaldolase) | | |
| thyA | Thymidylate Synthase | EP1108790 | AX121026 |
| | EC 2.1.1.45 | | AX127145 |
| | (thymidylate synthase) | | |
| tkt | Transketolase EC 2.2.1.1 (transketolase) | Ikeda et al., NCBI | AB023377 |
| tpi | Triose Phosphate Isomerase EC 5.3.1.1 (triose phosphate isomerase) | Eikmanns, Journal of Bacteriology 174:6076-6086 (1992) | X59403 |
| zwa1 | Cell Growth Factor 1 (growth factor 1) | EP1111062 | AX133781 |
| zwf | Glucose 6-Phosphate 1- | EP1108790 | AX127148 |
| | Dehydrogenase | | AX121827 |
| | EC 1.1.1.49 | WO0104325 | AX076272 |
| | (glucose 6-phosphate 1-dehydrogenase) | | |
| zwf A213T | Glucose 6-Phosphate 1-Dehydrogenase EC 1.1.1.49 (glucose 6-phosphate 1-dehydrogenase) amino acid exchange A213T | EP1108790 | |

**Table 5**

| Further gene sites for integration of open reading frames, genes and alleles of methionine production | | | |
|---|---|---|---|
| Gene name | Description of the coded enzyme or protein | Reference | Access Number |
| brnE | Transporter of | EP1096010 | AX137709 |
| | branched-chain amino acids | | AX137714 |
| | (branched-chain amino acid transporter) | | |
| brnF | Transporter of | EP1096010 | AX137709 |
| | branched-chain amino acids | | AX137714 |
| | (branched-chain amino acid transporter) | | |
| brnQ | Carrier protein of | Tauch et al., Archives of Microbiology 169(4):303-12 (1998) | M89931 |
| | branched-chain amino | | AX066841 |
| | acids | | AX127150 |
| | (branched-chain amino | W00100805 | |
| | acid transport system carrier protein) | EP1108790 | |
| ccpA1 | Catabolite Control | WO0100844 | AX065267 |
| | Protein | EP1108790 | AX127147 |
| | (catabolite control protein A1) | | |
| ccpA2 | Catabolite Control | WO0100844 | AX065267 |
| | Protein | EP1108790 | AX121594 |
| | (catabolite control protein A2) | | |
| citA | Sensor Kinase CitA (sensor kinase CitA) | EP1108790 | AX120161 |
| citB | Transcription Regulator CitB | EP1108790 | AX120163 |
| | (transcription regulator CitB) | | |
| citE | Citrate Lyase | WO0100844 | AX065421 |
| | EC 4.1.3.6 | EP1108790 | AX127146 |
| | (citrate lyase) | | |
| ddh | Diaminopimelate | Ishino et al., Nucleic Acids Research 15: 3917 (1987) | S07384 |
| | Dehydrogenase | | AX127152 |
| | EC 1.4.1.16 | | |
| | (diaminopimelate dehydrogenase) | EP1108790 | |
| gluA | Glutamate Transport ATP-binding Protein (glutamate transport ATP-binding protein) | Kronemeyer et al., Journal of Bacteriology 177(5):1152-8 (1995) | X81191 |
| gluB | Glutamate-binding Protein (glutamate-binding protein) | Kronemeyer et al., Journal of Bacteriology 177(5):1152-8 (1995) | X81191 |
| gluC | Glutamate Transport Permease | Kronemeyer et al., Journal of Bacteriology | X81191 |
| | (glutamate transport system permease) | 177(5):1152-8 (1995) | |
| gluD | Glutamate Transport Permease (glutamate transport system permease) | Kronemeyer et al., Journal of Bacteriology 177(5):1152-8 (1995) | X81191 |
| luxR | Transcription Regulator | WO0100842 | AX065953 |
| | LuxR | EP1108790 | AX123320 |
| | (transcription regulator LuxR) | | |
| luxS | Histidine Kinase LuxS | EP1108790 | AX123323 |
| | (histidine kinase LuxS) | | AX127153 |
| lysR1 | Transcription Regulator | EP1108790 | AX064673 |
| | LysR1 | | AX127144 |
| | (transcription regulator LysR1) | | |
| lysR2 | Transcription Activator | EP1108790 | AX123312 |
| | LysR2 | | |
| | (transcription regulator LysR2) | | |
| lysR3 | Transcription Regulator | WO0100842 | AX065957 |
| | LysR3 | EP1108790 | AX127150 |
| | (transcription regulator LysR3) | | |
| menE | O-Succinylbenzoic Acid | WO0100843 | AX064599 |
| | CoA Ligase | EP1108790 | AX064193 |
| | EC 6.2.1.26 | | AX127144 |
| | (O-succinylbenzoate CoA ligase) | | |
| metD | Transcription Regulator | EP1108790 | AX123327 |
| | MetD | | AX127153 |
| | (transcription regulator MetD) | | |
| metK | Methionine Adenosyl | WO0100843 | AX063959 |
| | Transferase | EP1108790 | AX127148 |
| | EC 2.5.1.6 (S-adenosylmethionine synthetase) | | |
| pck | Phosphoenol Pyruvate | WO0100844 | AJ269506 |
| | Carboxykinase (phosphoenol pyruvate carboxykinase) | | AX065053 |
| pgi | Glucose 6-Phosphate | EP1087015 | AX136015 |
| | Isomerase | EP1108790 | AX127146 |
| | EC 5.3.1.9 | | |
| | (glucose-6-phosphate isomerase) | | |
| poxB | Pyruvate Oxidase | WO0100844 | AX064959 |
| | EC 1.2.3.3 | EP1096013 | AX137665 |
| | (pyruvate oxidase) | | |
| zwa2 | Cell Growth Factor 2 | EP1106693 | AX113822 |
| | (growth factor 2) | EP1108790 | AX127146 |

A "copy of gene or allele of threonine production" is to be understood as meaning all the, preferably endogenous, genes or alleles of which enhancement/over-expression can have the effect of improving threonine production.

These include, inter alia, the following, genes: accBC, accDA, cstA, cysD, cysE, cysH, cysI, cysN, cysQ, dps, eno, fda, gap, gap2, gdh, gnd, hom, hom^{FBR}, lysC, lysC^{FBR}, msiK, opcA, oxyR, ppc, ppc^{FBR}, pgk, pknA, pknB, pknD, pknG, ppsA, ptsH, ptsI, ptsM, pyc, pyc P458S, sigC, sigD, sigE, sigH, sigM, tal, thyA, tkt, tpi, thrB, thrC, thrE, zwa1 zwf and zwf A213T. These are summarized and explained in Table 6. These include, in particular, the lysC^{FBR} alleles which code for a "feed back" resistant aspartate kinase (See Table 2) and the hom^{FBR} alleles which code for a "feed back" resistant homoserine dehydrogenase.

The at least third, optionally fourth or fifth copy of the gene of threonine production in question can be integrated at a site. The following open reading frames, genes or nucleotide sequences, inter alia, can be used for this: ccpA1, ccpA2, citA, citB, citE, ddh, gluA, gluB, gluC, gluD, glyA, ilvA, ilvBN, ilvC, ilvD, luxR, luxS, lysR1, lysR2, lysR3, mdh, menE, metA, metD, pck, poxB, sigB and zwa2. These are summarized and explained in Table 7. Intergenic regions in the chromosome, that is to say nucleotide sequences without a coding function, can furthermore be used. Finally, prophages or defective phages contained in the chromosome can be used for this.

**Table 6**

| Open reading frames, genes and alleles of threonine production | | | |
|---|---|---|---|
| Name | Description of the coded enzyme or protein | Reference | Access Number |
| accBC | Acyl-CoA Carboxylase EC 6.3.4.14 (acyl-CoA carboxylase) | Jäger et al. Archives of Microbiology (1996) 166:76-82 | U35023 |
| | | | AX123524 |
| | | EP1108790; | AX066441 |
| | | WO0100805 | |
| accDA | Acetyl-CoA Carboxylase | EP1055725 | |
| | EC 6.4.1.2 | EP1108790 | A121013 |
| | (acetyl-CoA carboxylase) | WO0100805 | AX066443 |
| cstA | Carbon Starvation Protein A | EP1108790 | AX120811 |
| | (carbon starvation protein A) | WO0100804 | AX066109 |
| cysD | Sulfate Adenylyltransferase sub-unit II | EP1108790 | AX123177 |
| | EC 2.7.7.4 | | |
| | (sulfate adenylyltransferase small chain) | | |
| cysE | Serine Acetyltransferase | EP1108790 | AX122902 |
| | EC 2.3.1.30 | WO0100843 | AX063961 |
| | (serine acetyltransferase) | | |
| cysH | 3'-Phosphoadenyl Sulfate Reductase | EP1108790 | AX123178 |
| | EC 1.8.99.4 | WO0100842 | AX066001 |
| | (3'-phosphoadenosine 5'-phosphosulfate reductase) | | |
| cysK | Cysteine Synthase | EP1108790 | AX122901 |
| | EC 4.2.99.8 | WO0100843 | AX063963 |
| | (cysteine synthase) | | |
| cysN | Sulfate Adenylyltransferase sub-unit I | EP1108790 | AX123176 |
| | EC 2.7.7.4 (sulfate adenylyltransferase) | | AX127152 |
| cysQ | Transport protein CysQ | EP1108790 | AX127145 |
| | (transporter cysQ) | WO0100805 | AX066423 |
| dps | DNA Protection Protein (protection during starvation protein) | EP1108790 | AX127153 |
| eno | Enolase | EP1108790 | AX127146 |
| | EC 4.2.1.11 | WO0100844 | AX064945 |
| | (enolase) | EP1090998 | AX136862 |
| | | Hermann et al., Electrophoresis 19:3217-3221 (1998) | |
| fda | Fructose Bisphosphate Aldolase EC 4.1.2.13 (fructose bisphosphate aldolase) | van der Osten et al., Molecular Microbiology 3:1625-1637 (1989) | X17313 |
| gap | Glyceraldehyde 3-Phosphate Dehydrogenase | EP1108790 | AX127148 |
| | EC 1.2.1.12 | WO0100844 | AX064941 |
| | (glyceraldehyde 3-phosphate dehydrogenase) | Eikmanns et al., Journal of Bacteriology 174:6076-6086(1992) | X59403 |
| gap2 | Glyceraldehyde 3-Phosphate Dehydrogenase | EP1108790 | AX127146 |
| | EC 1.2.1.12 | WO0100844 | AX064939 |
| | (glyceraldehyde 3-phosphate dehydrogenase 2) | | |
| gdh | Glutamate Dehydrogenase | EP1108790 | AX127150 |
| | EC 1.4.1.4 | WO0100844 | AX063811 |
| | (glutamate dehydrogenase) | Boermann et al., Molecular Microbiology 6:317-326 (1992) | X59404 |
| | | | X72855 |
| | | Guyonvarch et al, NCBI | |
| gnd | 6-Phosphogluconate Dehydrogenase | EP1108790 | AX127147 |
| | EC 1.1.1.44 | | AX121689 |
| | (6-phosphogluconate dehydrogenase) | WO0100844 | AX065125 |
| hom | Homoserine Dehydrogenase EC 1.1.1.3 (homoserine dehydrogenase) | Peoples et al., Molecular Microbiology 2:63-72 (1988) | Y00546 |
| hom^{FBR} | Homoserine Dehydrogenase feedback resistant (fbr) EC 1.1.1.3 (homoserine dehydrogenase fbr) | Reinscheid et al., Journal of Bacteriology 173:3228-30 (1991) | |
| lysC | Aspartate Kinase | EP1108790 | AX120365 |
| | EC 2.7.2.4 | WO0100844 | AX063743 |
| | (aspartate kinase) | Kalinowski et al., Molecular Microbiology 5:1197-204 (1991) | X57226 |
| lysC^{FBR} | Aspartate Kinase feedback resistent (fbr) EC 2.7.2.4 (aspartate kinase fbr) | see Table 2 | |
| msiK | Sugar Importer (multiple sugar import protein) | EP1108790 | AX120892 |
| opcA | Glucose 6-Phosphate Dehydrogenase (subunit of glucose 6-phosphate dehydrogenase) | WO0104325 | AX076272 |
| oxyR | Transcription Regulator | EP1108790 | AX122198 |
| | (transcriptional regulator) | | AX127149 |
| ppc^{FBR} | Phosphoenol Pyruvate Carboxylase | EP0723011 | |
| | feedback resistent | WO0100852 | |
| | EC 4.1.1.31 | | |
| | (phosphoenol pyruvate carboxylase feedback resistant) | | |
| ppc | Phosphoenol Pyruvate Carboxylase | EP1108790 | AX127148 |
| | EC 4.1.1.31 | | AX123554 |
| | (phosphoenol pyruvate carboxylase) | O'Reagan et al., Gene 77(2):237-251(1989) | M25819 |
| pgk | Phosphoglycerate Kinase | EP1108790 | AX121838 |
| | EC 2.7.2.3 | | AX127148 |
| | (phosphoglycerate kinase) | WO0100844 | AX064943 |
| | | Eikmanns, Journal of Bacteriology 174:6076-6086 (1992) | X59403 |
| pknA | Protein Kinase A | EP1108790 | AX120131 |
| | (protein kinase A) | | AX120085 |
| pknB | Protein Kinase B | EP1108790 | AX120130 |
| | (protein kinase B) | | AX120085 |
| pknD | Protein Kinase D | EP1108790 | AX127150 |
| | (protein kinase D) | | AX122469 |
| | | | AX122468 |
| pknG | Protein Kinase G | EP1108790 | AX127152 |
| | (protein kinase G) | | AX123109 |
| ppsA | Phosphoenol Pyruvate Synthase | EP1108790 | AX127144 |
| | EC 2.7.9.2 | | AX120700 |
| | (phosphoenol pyruvate synthase) | | AX122469 |
| ptsH | Phosphotransferase System Protein H | EP1108790 | AX122210 |
| | EC 2.7.1.69 | | AX127149 |
| | (phosphotransferase system component H) | WO0100844 | AX069154 |
| ptsI | Phosphotransferase System Enzyme I | EP1108790 | AX122206 |
| | EC 2.7.3.9 | | AX127149 |
| | (phosphotransferase system enzyme I) | | |
| ptsM | Glucose-specific Phosphotransferase | Lee et al., FEMS Microbiology Letters 119(1-2):137-145 (1994) | L18874 |
| | System Enzyme II EC 2.7.1.69 | | |
| | (glucose phosphotransferase-system enzyme II) | | |
| pyc | Pyruvate Carboxylase | WO9918228 | A97276 |
| | EC 6.4.1.1 (pyruvate carboxylase) | Peters-Wendisch et al., Microbiology 144:915-927 (1998) | Y09548 |
| pyc P458S | Pyruvate Carboxylase EC 6.4.1.1 (pyruvate carboxylase) amino acid exchange P458S | EP1108790 | |
| sigC | Sigma Factor C | EP1108790 | AX120368 |
| | EC 2.7.7.6 | | AX120085 |
| | (extracytoplasmic function alternative sigma factor C) | | |
| sigD | RNA Polymerase Sigma Factor D | EP1108790 | AX120753 |
| | EC 2.7.7.6 | | AX127144 |
| | (RNA polymerase sigma factor) | | |
| sigE | Sigma Factor E | EP1108790 | AX127146 |
| | EC 2.7.7.6 | | AX121325 |
| | (extracytoplasmic function alternative sigma factor E) | | |
| sigH | Sigma Factor H | EP1108790 | AX127145 |
| | EC 2.7.7.6 | | AX120939 |
| | (sigma factor SigH) | | |
| sigM | Sigma Factor M | EP1108790 | AX123500 |
| | EC 2.7.7.6 | | AX127153 |
| | (sigma factor SigM) | | |
| tal | Transaldolase | W00104325 | AX076272 |
| | EC 2.2.1.2 (transaldolase) | | |
| thrB | Homoserine Kinase EC 2.7.1.39 (homoserine kinase) | Peoples et al., Molecular Microbiology 2:63-72 (1988) | Y00546 |
| thrC | Threonine Synthase EC 4.2.99.2 (threonine synthase) | Han et al., Molecular Microbiology 4:1693-1702 (1990) | X56037 |
| thrE | Threonine Exporter (threonine export carrier) | EP1085091 | AX137526 |
| thyA | Thymidylate Synthase | EP1108790 | AX121026 |
| | EC 2.1.1.45 | | AX127145 |
| | (thymidylate synthase) | | |
| tkt | Transketolase EC 2.2.1.1 (transketolase) | Ikeda et al., NCBI | AB023377 |
| tpi | Triose Phosphate Isomerase EC 5.3.1.1 (triose phosphate isomerase) | Eikmanns, Journal of Bacteriology 174:6076-6086 (1992) | X59403 |
| zwa1 | Cell Growth Factor 1 (growth factor 1) | EP1111062 | AX133781 |
| zwf | Glucose 6-Phosphate 1-Dehydrogenase EC 1.1.1.49 (glucose 6-phosphate 1-dehydrogenase) | EP1108790 WO0104325 | |
| zwf | Glucose 6-Phosphate 1-Dehydrogenase | EP1108790 | AX127148 |
| A213T | EC 1.1.1.49 | | AX121827 |
| | (glucose 6-phosphate 1-dehydrogenase) amino acid exchange A213T | | AX076272 |

**Table 7**

| Further gene sites for integration of open reading frames, genes and alleles of threonine production | | | |
|---|---|---|---|
| Gene name | Description of the coded enzyme or protein | Reference | Access Number |
| ccpA1 | Catabolite Control | WO0100844 | AX065267 |
| | Protein | EP1108790 | AX127147 |
| | (catabolite control protein A1) | | |
| ccpA2 | Catabolite Control | WO0100844 | AX065267 |
| | Protein | EP1108790 | AX121594 |
| | (catabolite control protein A2) | | |
| citA | Sensor Kinase CitA | EP1108790 | AX120161 |
| | (sensor kinase CitA) | | |
| citB | Transcription Regulator | EP1108790 | AX120163 |
| | CitB | | |
| | (transcription regulator | | |
| | CitB) | | |
| citE | Citrate Lyase | WO0100844 | AX065421 |
| | EC 4.1.3.6 | EP1108790 | AX127146 |
| | (citrate lyase) | | |
| ddh | Diaminopimelate | Ishino et al., Nucleic Acids Research 15: 3917 (1987) | S07384 |
| | Dehydrogenase EC 1.4.1.16 | | AX127152 |
| | (diaminopimelate dehydrogenase) | EP1108790 | |
| gluA | Glutamate Transport ATP-binding Protein (glutamate transport ATP-binding protein) | Kronemeyer et al., Journal of Bacteriology 177(5):1152-8 (1995) | X81191 |
| gluB | Glutamate-binding Protein (glutamate-binding protein) | Kronemeyer et al., Journal of Bacteriology 177(5):1152-8 (1995) | X81191 |
| gluC | Glutamate Transport Permease (glutamate transport system permease) | Kronemeyer et al., Journal of Bacteriology 177(5):1152-8 (1995) | X81191 |
| gluD | Glutamate Transport Permease (glutamate transport system permease) | Kronemeyer et al., Journal of Bacteriology 177(5):1152-8 (1995) | X81191 |
| glyA | Glycine | W00100843 | AX063861 |
| | Hydroxymethyltransferase EC 2.1.2.1 | | AF327063 |
| | (glycine hydroxymethyltransferase) | | |
| ilvA | Threonine Dehydratase | Möckel et al., Journal of Bacteriology 174 (24), 8065-8072 (1992) | A47044 |
| | EC 4.2.1.16 | | L01508 |
| | (threonine dehydratase) | | AX127150 |
| | | EP1108790 | |
| ilvBN | Acetolactate Synthase | Keilhauer et al., Journal of Bacteriology, 175(17):5595-603 (1993) | A48648 |
| | EC 4.1.3.18 | | L09232 |
| | (acetolactate synthase) | EP1108790 | AX127147 |
| ilvC | Reductoisomerase | Keilhauer et al., Journal of Bacteriology 175(17):5595-603 (1993) | C48648 |
| | EC 1.1.1.86 | | AX127147 |
| | (ketol-acid | | |
| | reductoisomerase) | EP1108790 | |
| ilvD | Dihydroxy-acid Dehydratase EC 4.2.1.9 (dihydroxy-acid dehydratase) | EP1006189 | AX136925 |
| luxR | Transcription Regulator | WO0100842 | AX065953 |
| | LuxR (transcription regulator LuxR) | EP1108790 | AX123320 |
| luxS | Histidine Kinase LuxS | EP1108790 | AX123323 |
| | (histidine kinase LuxS) | | AX127153 |
| lysR1 | Transcription Regulator | EP1108790 | AX064673 |
| | LysR1 | | AX127144 |
| | (transcription regulator LysR1) | | |
| lysR2 | Transcription Activator | EP1108790 | AX123312 |
| | LysR2 | | |
| | (transcription regulator LysR2) | | |
| lysR3 | Transcription Regulator | WO0100842 | AX065957 |
| | LysR3 | EP1108790 | AX127150 |
| | (transcription regulator LysR3) | | |
| mdh | Malate Dehydrogenase EC 1.1.1.37 (malate dehydrogenase) | WO0100844 | AX064895 |
| menE | O-Succinylbenzoic Acid | WO0100843 | AX064599 |
| | CoA Ligase | EP1108790 | AX064193 |
| | EC 6.2.1.26 | | AX127144 |
| | (O-succinylbenzoate CoA ligase) | | |
| metA | Homoserine O- | Park et al., Molecular Cells 30;8(3):286-94 (1998) | AX063895 |
| | Acetyltransferase EC 2.3.1.31 | | AX127145 |
| | (homoserine O- | WO0100843 | |
| | acetyltransferase) | EP1108790 | |
| metD | Transcription Regulator | EP1108790 | AX123327 |
| | MetD | | AX127153 |
| | (transcription regulator MetD) | | |
| pck | Phosphoenol Pyruvate | WO0100844 | AJ269506 |
| | Carboxykinase | | AX065053 |
| | (phosphoenol pyruvate carboxykinase) | | |
| poxB | Pyruvate Oxidase | WO0100844 | AX064959 |
| | EC 1.2.3.3 | EP1096013 | AX137665 |
| | (pyruvate oxidase) | | |
| sigB | RNA Polymerase Transcription Factor (RNA polymerase transcription factor) | EP1108790 | A127149 |
| zwa2 | Cell Growth Factor 2 | EP1106693 | AX113822 |
| | (growth factor 2) | EP1108790 | AX127146 |

The description accordingly also provides a process for the production of coryneform bacteria which produce L-methionine and/or L-threonine, characterized in that
a) the nucleotide sequence of a desired ORF, gene or allele of methionine production or threonine production, optionally including the expression and/or regulation signals, is isolated
b) at least two copies of the nucleotide sequence of the ORF, gene or allele of methionine production or threonine production are arranged in a row, preferably in tandem arrangement
c) the nucleotide sequence obtained according to b) is incorporated in a vector which does not replicate or replicates to only a limited extent in coryneform bacteria,
d) the nucleotide sequence according to b) or c) is transferred into coryneform bacteria, and
e) coryneform bacteria which have at least two copies of the desired ORF, gene or allele of methionine or threonine production at the particular desired natural site instead of the singular copy of the ORF, gene or allele originally present are isolated, no nucleotide sequence which is capable of/enables episomal replication in microorganisms, no nucleotide sequence which is capable of/enables transposition and no nucleotide sequence which imparts resistance to antibiotics remaining at the particular natural site (locus), and optionally
f) at least a third copy of the open reading frame (ORF), gene or allele of methionine production or threonine production in question is introduced at a further gene site, no nucleotide sequence which is capable of/enables episomal replication in microorganisms, no nucleotide sequence which is capable of/enables transposition and no nucleotide sequence which imparts resistance to antibiotics remaining at the further gene site.

The description also provides coryneform bacteria, in particular of the genus Corynebacterium, which produce L-valine, characterized in that
a) instead of the singular copy of an open reading frame (ORF), a gene or allele of valine production naturally present at the particular desired site (locus), these have at least two copies of the said open reading frame (ORF), gene or allele, preferably in tandem arrangement, no nucleotide sequence which is capable of/enables episomal replication in microorganisms, no nucleotide sequence which is capable of/enables transposition and no nucleotide sequence which imparts resistance to antibiotics being present at the particular site, and in that these
b) optionally have at least a third copy of the open reading frame (ORF), gene or allele of valine production mentioned at a further gene site, no nucleotide sequence which is capable of/enables episomal replication in microorganisms, no nucleotide sequence which is capable of/enables transposition and no nucleotide sequence which imparts resistance to antibiotics being present at the further gene site.

The description also furthermore provides a process for the preparation of L-valine, which comprises the following steps:
a) fermentation of coryneform bacteria, in particular of the genus Corynebacterium, which
   i) instead of the singular copy of an open reading frame (ORF), gene or allele of valine production present at the particular desired site (locus), have at least two copies of the open reading frame (ORF), gene or allele in question, preferably in tandem arrangement, no nucleotide sequence which is capable of/enables episomal replication in microorganisms, no nucleotide sequence which is capable of/enables transposition and no nucleotide sequence which imparts resistance to antibiotics being present at the particular site, and
   ii) optionally have at least a third copy of the open reading frame (ORF), gene or allele of valine production in question at a further gene site, no nucleotide sequence which is capable of/enables episomal replication in microorganisms, no nucleotide sequence which is capable of/enables transposition and no nucleotide sequence which imparts resistance to antibiotics being present at the further gene site,
      under conditions which allow expression of the said open reading frames (ORFs), genes or alleles,
b) concentration of the L-valine in the fermentation broth,
c) isolation of the L-valine from the fermentation broth, optionally
d) with constituents from the fermentation broth and/or the biomass to the extent of > (greater than) 0 to 100%.

A "copy of gene of valine production" is to be understood as meaning all the, preferably endogenous, genes or alleles of which enhancement/over-expression can have the effect of improving valine production.

These include, inter alia, the following genes: brnE, brnF, brnEF, cstA, cysD, dps, eno, fda, gap, gap2, gdh, ilvB, ilvN, ilvBN, ilvC, ilvD, ilvE msiK, pgk, ptsH, ptsI, ptsM, sigC, sigD, sigE, sigH, sigM, tpi and zwa1. These are summarized and explained in Table 8. These include in particular the acetolactate synthase ilvBN alleles which code for a valine-resistant.

The at least third, optionally fourth or fifth copy of the gene of valine production in question can be integrated at a further site. The following genes, inter alia, can be used for this: aecD, ccpA1 ccpA2, citA, citB, citE, ddh, gluA, gluB, gluC, gluD, glyA, ilvA, luxR, lysR1, lysR2, lysR3, panB, panC, poxB and zwa2. These are summarized and explained in Table 9. Intergenic regions in the chromosome, that is to say nucleotide sequences without a coding function, can furthermore be used. Finally, prophages or defective phages contained in the chromosome can be used for this.

**Table 8**

| Open reading frames, genes and alleles of valine production | | | |
|---|---|---|---|
| Name | Description of the coded enzyme or protein | Reference | Access Number |
| brnEF | Export of branched-chain amino acids | EP1096010 | |
| | (branched chain amino acid export) | Kennerknecht et al., NCBI | AF454053 |
| cstA | Carbon Starvation Protein A | EP1108790 | AX120811 |
| | (carbon starvation protein A) | WO0100804 | AX066109 |
| dps | DNA Protection Protein (protection during starvation protein) | EP1108790 | AX127153 |
| eno | Enolase | EP1108790 | AX127146 |
| | EC 4.2.1.11 | WO0100844 | AX064945 |
| | (enolase) | EP1090998 | AX136862 |
| | | Hermann et al., Electrophoresis 19:3217-3221 (1998) | |
| fda | Fructose Bisphosphate Aldolase EC 4.1.2.13 (fructose bisphosphate aldolase) | van der Osten et al., Molecular Microbiology 3:1625-1637 (1989) | X17313 |
| gap | Glyceraldehyde 3-Phosphate | EP1108790 | AX127148 |
| | Dehydrogenase | WO0100844 | AX064941 |
| | EC 1.2.1.12 (glyceraldehyde 3-phosphate dehydrogenase) | Eikmanns et al., Journal of Bacteriology 174:6076-6086(1992) | X59403 |
| gap2 | Glyceraldehyde 3-Phosphate | EP1108790 | AX127146 |
| | Dehydrogenase | WO0100844 | AX064939 |
| | EC 1.2.1.12 | | |
| | (glyceraldehyde 3-phosphate dehydrogenase 2) | | |
| gdh | Glutamate Dehydrogenase | EP1108790 | AX127150 |
| | EC 1.4.1.4 | WO0100844 | AX063811 |
| | (glutamate dehydrogenase) | Boermann et al., Molecular Microbiology 6:317-326 (1992); | X59404 |
| | | Guyonvarch et al., NCBI | X72855 |
| ilvBN | Acetolactate Synthase EC 4.1.3.18 (acetolactate synthase) | Keilhauer et al., Journal of Bacteriology 175(17):5595-603 (1993) | L09232 |
| | | EP1108790 | AX127147 |
| ilvC | Isomeroreductase | Keilhauer et al., Journal of | C48648 |
| | EC 1.1.1.86 | | AX127147 |
| | (acetohydroxy acid isomeroreductase) | Bacteriology 175(17):5595-603 (1993) EP1108790 | |
| ilvD | Dihydroxy-acid Dehydratase EC 4.2.1.9 (dihydroxy acid dehydratase) | EP1006189 | AX136925 |
| ilvE | Transaminase B | EP1108790 | AX127150 |
| | EC 2.6.1.42 | | AX122498 |
| | (transaminase B) | | |
| msiK | Sugar Importer | EP1108790 | AX120892 |
| | (multiple sugar import protein) | | |
| pgk | Phosphoglycerate Kinase | EP1108790 | AX121838 |
| | EC 2.7.2.3 | | AX127148 |
| | (phosphoglycerate kinase) | WO0100844 | AX064943 |
| | | Eikmanns, Journal of Bacteriology 174:6076-6086 (1992) | X59403 |
| ptsH | Phosphotransferase System Protein H | EP1108790 | AX122210 |
| | EC 2.7.1.69 | | AX127149 |
| | (phosphotransferase system component H) | WO0100844 | AX069154 |
| ptsI | Phosphotransferase System Enzyme I | EP1108790 | AX122206 |
| | EC 2.7.3.9 | | AX127149 |
| | (phosphotransferase system enzyme I) | | |
| ptsM | Glucose-specific Phosphotransferase System Enzyme II EC 2.7.1.69 (glucose phosphotransferase-system enzyme II) | Lee et al., FEMS Microbiology Letters 119(1-2):137-145 (1994) | L18874 |
| sigC | Sigma Factor C | EP1108790 | AX120368 |
| | EC 2.7.7.6 | | AX120085 |
| | (extracytoplasmic function alternative sigma factor C) | | |
| sigD | RNA Polymerase Sigma Factor D | EP1108790 | AX120753 |
| | EC 2.7.7.6 | | AX127144 |
| | (RNA polymerase sigma factor) | | |
| sigE | Sigma Factor E | EP1108790 | AX127146 |
| | EC 2.7.7.6 | | AX121325 |
| | (extracytoplasmic function alternative sigma factor E) | | |
| sigH | Sigma Factor H | EP1108790 | AX127145 |
| | EC 2.7.7.6 | | AX120939 |
| | (sigma factor SigH) | | |
| sigM | Sigma Factor M | EP1108790 | AX123500 |
| | EC 2.7.7.6 | | AX127153 |
| | (sigma factor SigM) | | |
| tpi | Triose Phosphate Isomerase | Eikmanns, Journal of Bacteriology 174:6076-6086 (1992) | X59403 |
| | EC 5.3.1.1 | | |
| | (triose phosphate isomerase) | | |
| zwa1 | Cell Growth Factor 1 (growth factor 1) | EP1111062 | AX133781 |

**Table 9**

| Further gene sites for integration of open reading frames, genes and alleles of valine production | | | |
|---|---|---|---|
| Gene name | Description of the coded enzyme or protein | Reference | Access Number |
| aecD | beta C-S Lyase | Rossol et al., Journal of Bacteriology 174(9):2968-77 (1992) | M89931 |
| | EC 2.6.1.1 | | |
| | (beta C-S lyase) | | |
| ccpA1 | Catabolite Control | WO0100844 | AX065267 |
| | Protein | EP1108790 | AX127147 |
| | (catabolite control protein A1) | | |
| ccpA2 | Catabolite Control | WO0100844 | AX065267 |
| | Protein | EP1108790 | AX121594 |
| | (catabolite control protein A2) | | |
| citA | Sensor Kinase CitA | EP1108790 | AX120161 |
| | (sensor kinase CitA) | | |
| citB | Transcription Regulator | EP1108790 | AX120163 |
| | CitB | | |
| | (transcription regulator | | |
| | CitB) | | |
| citE | Citrate Lyase | WO0100844 | AX065421 |
| | EC 4.1.3.6 | EP1108790 | AX127146 |
| | (citrate lyase) | | |
| ddh | Diaminopimelate | Ishino et al., Nucleic Acids Research 15: 3917 (1987) | S07384 |
| | Dehydrogenase EC 1.4.1.16 | | AX127152 |
| | (diaminopimelate dehydrogenase) | EP1108790 | |
| gluA | Glutamate Transport ATP-binding Protein (glutamate transport ATP-binding protein) | Kronemeyer et al., Journal of Bacteriology 177(5):1152-8 (1995) | X81191 |
| gluB | Glutamate-binding Protein (glutamate-binding protein) | Kronemeyer et al., Journal of Bacteriology 177(5):1152-8 (1995) | X81191 |
| gluC | Glutamate Transport Permease (glutamate transport system permease) | Kronemeyer et al., Journal of Bacteriology 177(5):1152-8 (1995) | X81191 |
| gluD | Glutamate Transport Permease (glutamate transport system permease) | Kronemeyer et al., Journal of Bacteriology 177(5):1152-8 (1995) | X81191 |
| glyA | Glycine | WO0100843 | AX063861 |
| | Hydroxymethyltransferase | | AF327063 |
| | EC 2.1.2.1 | | |
| | (glycine hydroxymethyltransferase) | | |
| ilvA | Threonine Dehydratase | Möckel et al., Journal of Bacteriology 174 (24), 8065-8072 (1992) | A47044 |
| | EC 4.2.1.16 | | L01508 |
| | (threonine dehydratase) | | AX127150 |
| | | EP1108790 | |
| luxR | Transcription Regulator | WO0100842 | AX065953 |
| | LuxR | EP1108790 | AX123320 |
| | (transcription regulator LuxR) | | |
| lysR1 | Transcription Regulator | EP1108790 | AX064673 |
| | LysR1 | | AX127144 |
| | (transcription regulator LysR1) | | |
| lysR2 | Transcription Activator | EP1108790 | AX123312 |
| | LysR2 | | |
| | (transcription regulator LysR2) | | |
| lysR3 | Transcription Regulator | WO0100842 | AX065957 |
| | LysR3 | EP1108790 | AX127150 |
| | (transcription regulator LysR3) | | |
| panB | Ketopantoate | US6177264 | X96580 |
| | Hydroxymethyltransferase EC 2. 1. 2. 11 | | |
| | (ketopantoate hydroxymethyltransferase) | | |
| panC | Pantothenate Synthetase | US6177264 | X96580 |
| | EC 6.3.2.1 (pantothenate synthetase) | | |
| poxB | Pyruvate Oxidase | WO0100844 | AX064959 |
| | EC 1.2.3.3 | EP1096013 | AX137665 |
| | (pyruvate oxidase) | | |
| zwa2 | Cell Growth Factor 2 | EP1106693 | AX113822 |
| | (growth factor 2) | EP1108790 | AX127146 |

The description accordingly also provides a process for the production of coryneform bacteria which produce L-valine, characterized in that
a) the nucleotide sequence of a desired ORF, gene or allele of valine production, optionally including the expression and/or regulation signals, is isolated
b) at least two copies of the nucleotide sequence of the ORF, gene or allele of valine production are arranged in a row, preferably in tandem arrangement
c) the nucleotide sequence obtained according to b) is incorporated in a vector which does not replicate or replicates to only a limited extent in coryneform bacteria,
d) the nucleotide sequence according to b) or c) is transferred into coryneform bacteria, and
e) coryneform bacteria which have at least two copies of the desired open ORF, gene or allele of valine production at the particular desired natural site instead of the singular copy of the ORF, gene or allele originally present are isolated, no nucleotide sequence which is capable of/enables episomal replication in microorganisms, no nucleotide sequence which is capable of/enables transposition and no nucleotide sequence which imparts resistance to antibiotics remaining at the particular natural site (locus), and optionally
f) at least a third copy of the open reading frame (ORF), gene or allele of valine production in question is introduced at a further gene site, no nucleotide sequence which is capable of/enables episomal replication in microorganisms, no nucleotide sequence which is capable of/enables transposition and no nucleotide sequence which imparts resistance to antibiotics remaining at the further gene site.

During work on the present invention, it was possible to incorporate two copies, arranged in tandem, of an lysC^{FBR} allele at the lysC gene site of Corynebacterium glutamicum such that no nucleotide sequence which is capable of/enables episomal replication in microorganisms, no nucleotide sequence which is capable of/enables transposition and no nucleotide sequence which imparts resistance to antibiotics remain at the lysC gene site. Such a strain is, for example, the strain DSM13992lysC^{FBR}: :lysC^{FBR}.

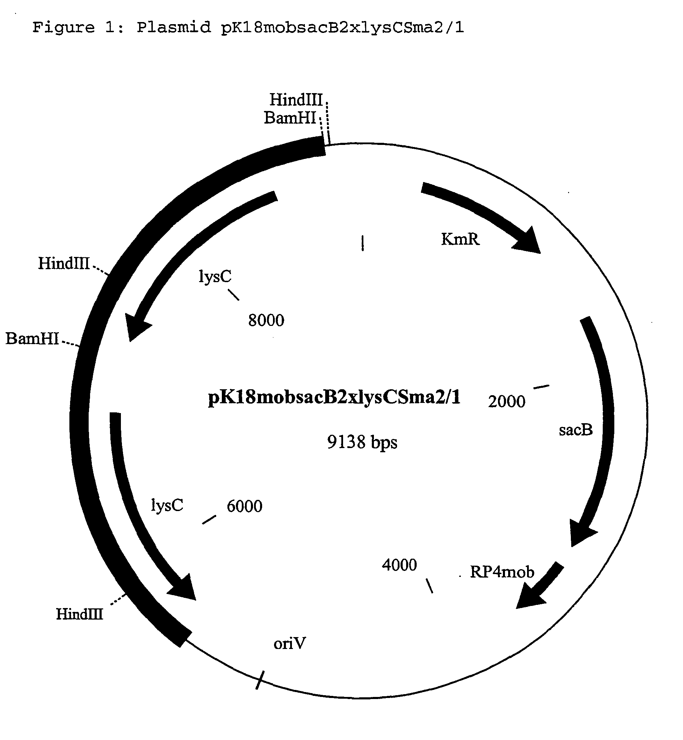

The plasmid pK18mobsacB2xlysCSma2/1, with the aid of which two copies of an lysC^{FBR} allele can be incorporated into the lysC gene site of Corynebacterium glutamicum, is shown in Figure 1.

During work on the present invention, it was furthermore possible to incorporate two copies, arranged in tandem, of the lysE gene at the lysE gene site of Corynebacterium glutamicum such that no nucleotide sequence which is capable of/enables episomal replication in microorganisms, no nucleotide sequence which is capable of/enables transposition and no nucleotide sequence which imparts resistance to antibiotics remained at the lysE gene site. Such a strain is, for example, the strain ATCC21513_17lysE::lysE.

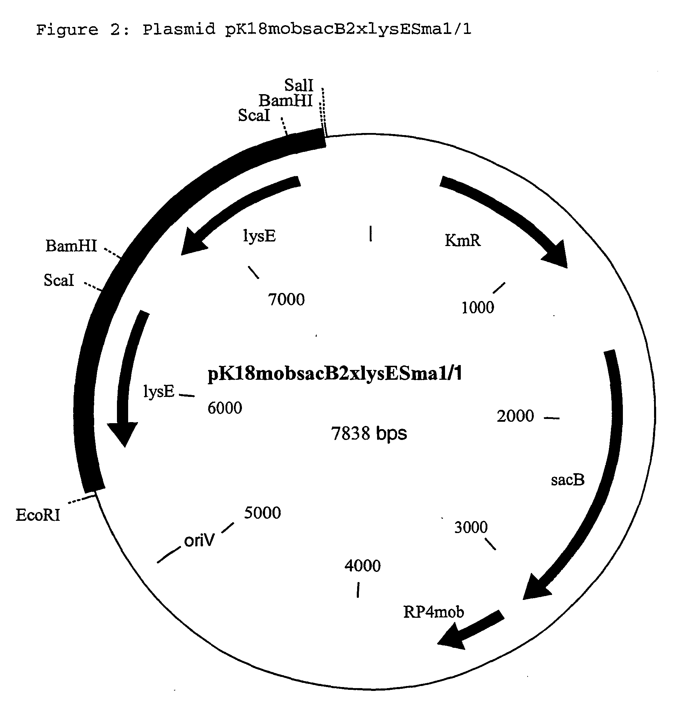

A plasmid with the aid of which two copies of an lysE gene can be incorporated into the lysE gene site of Corynebacterium glutamicum is shown in Figure 2. It carries the name pK18mobsacB2xlysESma1/1.

During work on the present invention, finally, it was possible to incorporate two copies, arranged in tandem, of the zwa1 gene at the zwa1 gene site of Corynebacterium glutamicum such that no nucleotide sequence which is capable of/enables episomal replication in microorganisms, no nucleotide sequence which is capable of/enables transposition and no nucleotide sequence which imparts resistance to antibiotics remained at the zwa1 gene site. Such a strain is, for example, the strain ATCC21513_17zwa1::zwa1.

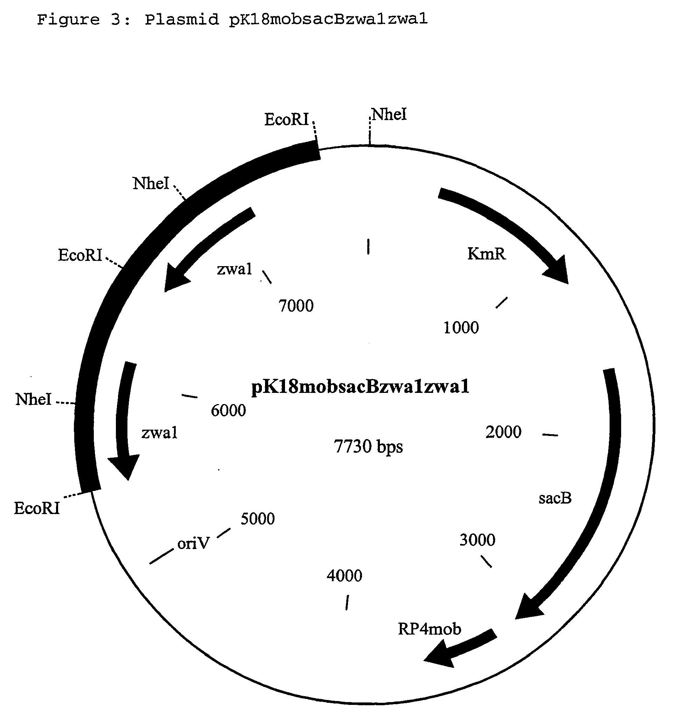

A plasmid with the aid of which two copies of a zwa1 gene can be incorporated into the zwa1 gene site of Corynebacterium glutamicum is shown in Figure 3. It carries the name pK18mobsacBzwa1zwa1.

The coryneform bacteria produced according to the invention can be cultured continuously or discontinuously in the batch process (batch culture) or in the fed batch (feed process) or repeated fed batch process (repetitive feed process) for the purpose of production of chemical compounds. A summary of known culture methods is described in the textbook by Chmiel (Bioprozesstechnik 1. Einführung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/Wiesbaden, 1994)).

The culture medium to be used must meet the requirements of the particular strains in a suitable manner. Descriptions of culture media for various microorganisms are contained in the handbook "Manual of Methods for General Bacteriology" of the American Society for Bacteriology (Washington D.C., USA, 1981).

Sugars and carbohydrates, such as e.g. glucose, sucrose, lactose, fructose, maltose, molasses, starch and cellulose, oils and fats, such as e.g. soya oil, sunflower oil, groundnut oil and coconut fat, fatty acids, such as e.g. palmitic acid, stearic acid and linoleic acid, alcohols, such as e.g. glycerol and ethanol, and organic acids, such as e.g. acetic acid or lactic acid, can be used as the source of carbon. These substances can be used individually or as a mixture.

Organic nitrogen-containing compounds, such as peptones, yeast extract, meat extract, malt extract, corn steep liquor, soya bean flour and urea, or inorganic compounds, such as ammonium sulfate, ammonium chloride, ammonium phosphate, ammonium carbonate and ammonium nitrate, can be used as the source of nitrogen. The sources of nitrogen can be used individually or as a mixture.

Phosphoric acid, potassium dihydrogen phosphate or dipotassium hydrogen phosphate or the corresponding sodium-containing salts can be used as the source of phosphorus. The culture medium must furthermore comprise salts of metals, such as e.g. magnesium sulfate or iron sulfate, which are necessary for growth. Finally, essential growth substances, such as amino acids and vitamins, can be employed in addition to the above-mentioned substances. Suitable precursors can moreover be added to the culture medium. The starting substances mentioned can be added to the culture in the form of a single batch, or can be fed in during the culture in a suitable manner.

Basic compounds, such as sodium hydroxide, potassium hydroxide, ammonia or aqueous ammonia, or acid compounds, such as phosphoric acid or sulfuric acid, can be employed in a suitable manner to control the pH of the culture. Antifoams, such as e.g. fatty acid polyglycol esters, can be employed to control the development of foam. Suitable substances having a selective action, such as e.g. antibiotics, can be added to the medium to maintain the stability of plasmids. To maintain aerobic conditions, oxygen or oxygen-containing gas mixtures, such as e.g. air, are introduced into the culture. The temperature of the culture is usually 20°C to 45ºC, and preferably 25ºC to 40ºC. Culturing is continued until a maximum of the desired chemical compound has formed. This target is usually reached within 10 hours to 160 hours.

It has been found that the coryneform bacteria according to the invention, in particular the coryneform bacteria which produce L-lysine, have an unexpectedly high stability. They were stable for at least 10-20, 20-30, 30-40, 40-50, preferably at least 50-60, 60-70, 70-80 and 80-90 generations or cell division cycles.

The following microorganisms have been deposited:

The Corynebacterium glutamicum strain
DSM13992lysC^{FBR}: :lysC^{FBR} was deposited in the form of a pure culture on 5th June 2002 under number DSM15036 at the Deutsche Sammlung für Mikroorganismen und Zellkulturen (DSMZ, Braunschweig, Germany) in accordance with the Budapest Treaty.

The plasmid pK18mobsacB2xlysCSma2/1 was deposited in the form of a pure culture of the strain E. coli DH5αmcr/pK18mobsacB2xlysCSma2/1 (= DH5alphamcr/pK18mobsacB2xlysCSma2/1) on 20th April 2001 under number DSM14244 at the Deutsche Sammlung für Mikroorganismen und Zellkulturen (DSMZ, Braunschweig, Germany) in accordance with the Budapest Treaty.

The Corynebacterium glutamicum strain ATCC21513_17lysE::lysE was deposited in the form of a pure culture on 5th June 2002 under number DSM15037 at the Deutsche Sammlung für Mikroorganismen und Zellkulturen (DSMZ, Braunschweig, Germany) in accordance with the Budapest Treaty.

The Corynebacterium glutamicum strain ATCC21513_17zwa1::zwa1 was deposited in the form of a pure culture on 5th June 2002 under number DSM15038 at the Deutsche Sammlung für Mikroorganismen und Zellkulturen (DSMZ, Braunschweig, Germany) in accordance with the Budapest Treaty.

### Example 1

Generation of a tandem duplication of the lysC^{FBR} allele lysC T311I in the chromosome of Corynebacterium glutamicum

### 1.1. Construction of the tandem vector pK18mobsacB2×lysCSma2/1

From the Corynebacterium glutamicum strain DSM13994, chromosomal DNA is isolated by the conventional methods (Eikmanns et al., Microbiology 140: 1817 - 1828 (1994)).

The strain DSM13994 was produced by multiple, non-directed mutagenesis, selection and mutant selection from C. glutamicum ATCC13032. The strain is resistant to the lysine analogue S-(2-aminoethyl)-L-cysteine and has a feed back-resistant aspartate kinase which is insensitive to inhibition by a mixture of lysine and threonine (in each case 25 mM). The nucleotide sequence of the lysC^{FBR} allele is shown as SEQ ID NO:3. It is also called lysC T311I in the following. The amino acid sequence of the aspartate kinase protein coded is shown as SEQ ID NO:4. A pure culture of this strain was deposited on 16th January 2001 at the Deutsche Sammlung für Mikroorganismen und Zellkulturen (DSMZ, Braunschweig, Germany) in accordance with the Budapest Treaty.

With the aid of the polymerase chain reaction, a DNA section which carries the lysC gene or allele is amplified. On the basis of the sequence of the lysC gene known for C. glutamicum (Kalinowski et al., Molecular Microbiology, 5 (5), 1197 - 1204(1991); Accession Number X57226), the following primer oligonucleotides were chosen for the PCR:
lysC1beg (SEQ ID No: 15):
   5' TA(G GAT CC)T CCG GTG TCT GAC CAC GGT G 3'
lysC2end: (SEQ ID NO: 16):
   5' AC(G GAT CC)G CTG GGA AAT TGC GCT CTT CC 3'

The primers shown are synthesized by MWG Biotech and the PCR reaction is carried out by the standard PCR method of Innis et al. (PCR Protocols. A Guide to Methods and Applications, 1990, Academic Press). The primers allow amplification of a DNA section of approx. 1.7 kb in length, which carries the lysC gene or allele. The primers moreover contain the sequence for a cleavage site of the restriction endonuclease BamHI, which is marked by parentheses in the nucleotide sequence shown above.

The amplified DNA fragment of approx. 1.7 kb in length which carries the lysC^{FBR} allele lysC T311I of the strain DSM13994 is identified by electrophoresis in a 0.8% agarose gel, isolated from the gel and purified by conventional methods (QIAquick Gel Extraction Kit, Qiagen, Hilden).

Ligation of the fragment is then carried out by means of the Topo TA Cloning Kit (Invitrogen, Leek, The Netherlands, Cat. Number K4600-01) in the vector pCRII-TOPO. The ligation batch is transformed in the E. coli strain TOP10 (Invitrogen, Leek, The Netherlands). Selection of plasmid-carrying cells is made by plating out the transformation batch on kanamycin (50 mg/l)-containing LB agar with X-Gal (5-bromo-4-chloro-3-indolyl β-D-galactopyranoside, 64 mg/l).

The plasmid obtained is checked by means of restriction cleavage, after isolation of the DNA, and identified in agarose gel. The resulting plasmid is called pCRIITOPOlysC.

The nucleotide sequence of the amplified DNA fragment or PCR product is determined by the dideoxy chain termination method of Sanger et al. (Proceedings of the National Academy of Sciences USA, 74:5463-5467 (1977)) using the "ABI Prism 377" sequencing apparatus of PE Applied Biosystems (Weiterstadt, Germany). The sequence of the coding region of the PCR product is shown in SEQ ID No:3. The amino acid sequence of the associated aspartate kinase protein is shown in SEQ ID NO:4.

The base thymine is found at position 932 of the nucleotide sequence of the coding region of the lysC^{FBR} allele of strain DSM13994 (SEQ ID NO:3). The base cytosine is found at the corresponding position of the wild-type gene (SEQ ID NO:1).

The amino acid isoleucine is found at position 311 of the amino acid sequence of the aspartate kinase protein of strain DSM13994 (SEQ ID No:4). The amino acid threonine is found at the corresponding position of the wild-type protein (SEQ ID No:2).

The lysC allele, which contains the base thymine at position 932 of the coding region and accordingly codes for an aspartate kinase protein which contains the amino acid isoleucine at position 311 of the amino acid sequence, is called the lysC^{FBR} allele lysC T311I in the following.

The plasmid pCRIITOPOlysC, which carries the lysC^{FBR} allele lysC T311I, was deposited in the form of a pure culture of the strain E. coli TOP 10/pCRIITOPOlysC under number DSM14242 on 20th April 2001 at the Deutsche Sammlung für Mikroorganismen und Zellkulturen (DSMZ = German Collection of Microorganisms and Cell Cultures, Braunschweig, Germany) in accordance with the Budapest Treaty.

Plasmid DNA was isolated from the strain DSM14242, which carries the plasmid pCRIITOPOlysC, and cleaved with the restriction enzyme BamHI (Amersham-Pharmacia, Freiburg, Germany), after separation in an agarose gel (0.8%) the lysC^{FBR}-containing DNA fragment approx. 1.7 kb long is isolated from the agarose gel with the aid of the QIAquick Gel Extraction Kit (Qiagen, Hilden, Germany), and the overhanging ends are completed with Klenow polymerase (Boehringer Mannheim) and employed for ligation with the mobilizable cloning vector pK18mobsacB described by Schäfer et al., Gene, 14, 69-73 (1994). This is cleaved beforehand with the restriction enzyme SmaI and dephosphorylated with alkaline phosphatase (Alkaline Phosphatase, Boehringer Mannheim), mixed with the lysC^{FBR}-containing fragment of approx. 1.7 kb and the mixture is treated with T4 DNA Ligase (Amersham-Pharmacia, Freiburg, Germany).

The E. coli strain DH5α (Grant et al.; Proceedings of the National Academy of Sciences USA, 87 (1990) 4645-4649) is then transformed with the ligation batch (Hanahan, In. DNA Cloning. A Practical Approach. Vol. 1, ILR-Press, Cold Spring Harbor, New York, 1989). Selection of plasmid-carrying cells is made by plating out the transformation batch on LB agar (Sambrook et al., Molecular Cloning: A Laboratory Manual. 2nd Ed., Cold Spring Harbor, New York, 1989), which was supplemented with 25 mg/l kanamycin.

Plasmid DNA is isolated from a transformant with the aid of the QIAprep Spin Miniprep Kit from Qiagen and checked by restriction cleavage with the enzyme HindIII and subsequent agarose gel electrophoresis. The plasmid is called pK18mobsacB1xlysCSma2.

In a second step, the plasmid pCRII-TOPOlysC is in turn cleaved with the restriction enzyme BamHI (Amersham-Pharmacia, Freiburg, Germany), after separation in an agarose gel (0.8%) the lysC^{FBR}-containing fragment of approx. 1.7 kb was isolated from the agarose gel with the aid of the QIAquick Gel Extraction Kit (Qiagen, Hilden, Germany) and employed for ligation with the vector pK18mobsacB1xlysCSma2 described in this Example. This is cleaved beforehand with the restriction enzyme BamHI and dephosphorylated with alkaline phosphatase (Alkaline Phosphatase, Boehringer Mannheim), mixed with the lysC^{FBR}-containing fragment of approx. 1.7 kb and the mixture is treated with T4 DNA Ligase (Amersham-Pharmacia, Freiburg, Germany).

The E. coli strain DH5α (Grant et al.; Proceedings of the National Academy of Sciences USA, 87 (1990) 4645-4649) is then transformed with the ligation batch (Hanahan, In. DNA Cloning. A Practical Approach. Vol. 1, ILR-Press, Cold Spring Harbor, New York, 1989). Selection of plasmid-carrying cells is made by plating out the transformation batch on LB agar (Sambrook et al., Molecular Cloning: A Laboratory Manual. 2nd Ed., Cold Spring Harbor, New York, 1989), which was supplemented with 25 mg/l kanamycin.

Plasmid DNA is isolated from a transformant with the aid of the QIAprep Spin Miniprep Kit from Qiagen and checked by restriction cleavage with the enzyme HindIII and subsequent agarose gel electrophoresis. The plasmid is called pK18mobsacB2xlysCSma2/1. A map of the plasmid is shown in Figure 1.

The plasmid pK18mobsacB2xlysCSma2/1 was deposited in the form of a pure culture of the strain E. coli DH5αmcr/pK18mobsacB2xlysCSma2/1 (= DH5alphamcr/pK18mobsacB2xlysCSma2/1) on 20th April 2001 under number DSM14244 at the Deutsche Sammlung für Mikroorganismen und Zellkulturen (DSMZ, Braunschweig, Germany) in accordance with the Budapest Treaty.

### 1.2. Generation of a tandem duplication of the lysC^{FBR} allele lysC T311I in C. glutamicum strain DSM13992

The vector pK18mobsacB2xlysCSma2/1 mentioned in Example 1.1 is transferred by a modified protocol of Schäfer et al. (1990 Journal of Microbiology 172: 1663-1666) into the C. glutamicum strain DSM13992.

The Corynebacterium glutamicum strain DSM13992 was produced by multiple, non-directed mutagenesis, selection and mutant selection from C. glutamicum ATCC13032. The strain is resistant to the antibiotic streptomycin and phenotypically resistant to the lysine analogue S-(2-aminoethyl)-L-cysteine. However, the strain has a wild-type aspartate kinase (see SEQ ID NO:1 and 2), which is sensitive to inhibition by a mixture of lysine and threonine (in each case 25 mM). A pure culture of this strain was deposited on 16th January 2001 at the Deutsche Sammlung für Mikroorganismen und Zellkulturen (DSMZ, Braunschweig, Germany) in accordance with the Budapest Treaty.

The vector pK18mobsacB2xlysCSma2/1 cannot replicate independently in DSM13992 and is retained in the cell only if it has integrated into the chromosome.

Selection of clones with integrated pK18mobsacB2xlysCSma2/1 is carried out by plating out the conjugation batch on LB agar (Sambrook et al., Molecular Cloning: A Laboratory Manual. 2nd Ed., Cold Spring Harbor, New York, 1989), which was supplemented with 15 mg/l kanamycin and 50 mg/l nalidixic acid. Clones which have grown on are plated out on LB agar plates with 25 mg/l kanamycin and incubated for 16 hours at 33°C. To achieve excision of the plasmid with only one copy of the lysC gene, the clones are cultured on LB agar with 10% sucrose, after incubation for 16 hours in LB liquid medium. The plasmid pK18mobsacB contains a copy of the sacB gene, which converts sucrose into levan sucrase, which is toxic to C. glutamicum.

Only those clones in which the pK18mobsacB2xlysCSma2/1 integrated has been excised again therefore grow on LB agar with sucrose. Approximately 40 to 50 colonies are tested for the phenotype "growth in the presence of sucrose" and "non-growth in the presence of kanamycin". During the excision, either two copies of the lysC gene or only one can be excised together with the plasmid.

To demonstrate that two copies of lysC have remained in the chromosome, approximately 20 colonies which show the phenotype "growth in the presence of sucrose" and "non-growth in the presence of kanamycin" are investigated with the aid of the polymerase chain reaction by the standard PCR method of Innis et al. (PCR Protocols. A Guide to Methods and Applications, 1990, Academic Press). A DNA fragment which carries the lysC gene and surrounding regions is amplified here from the chromosomal DNA of the colonies. The following primer oligonucleotides are chosen for the PCR.
lysCK1 (SEQ ID NO: 5):
5' TCG GTG TCA TCA GAG CAT TG 3'
lysCK2 (SEQ ID NO: 6):
5' TCG GTT GCC TGA GTA ATG TC 3'

The primers allow amplification of a DNA fragment approx. 1.9 kb in size in control clones with the original lysC locus. In clones with a second copy of the lysC gene in the chromosome at the lysC locus, DNA fragments with a size of approx. 3.6 kb are amplified.

The amplified DNA fragments are identified by means of electrophoresis in a 0.8% agarose gel. On the basis of the amplified fragment length, a distinction was made between clones with one chromosomal lysC gene copy and clones with two chromosomal lysC gene copies.

10 clones with two complete copies of the lysC gene on the chromosome are investigated with the aid of the LightCycler of Roche Diagnostics (Mannheim, Germany) in order to demonstrate whether the two copies are lysC^{FBR} alleles with the mutation lysC T311I or whether the original wild-type lysC is present alongside an lysC^{FBR} allele lysC T311I. The LightCycler is a combined apparatus of Thermocycler and flourimeter.

A DNA section approx. 500 bp in length which contains the mutation site is amplified in the first phase by means of a PCR (Innis et al., PCR Protocols. A Guide to Methods and Applications, 1990, Academic Press) using the following primer oligonucleotides.
LC-lysC1-fbr (SEQ ID No: 7):
5` aaccgttctgggtatttccg 3'
LC-lysC2-fbr (SEQ ID No: 8):
5` tccatgaactctgcggtaac 3'

In the second phase, with two additional oligonucleotides of different lengths and marked with different fluorescent dyestuffs (Lightcycler(LC)-Red640 and fluorescein), which hybridize in the region of the mutation site, the presence of the mutation is detected with the aid of the "Fluorescence Resonance Energy Transfer" method (FRET) using a melting curve analysis (Lay et al., Clinical Chemistry, 43:2262-2267 (1997)).
lysC311-C (SEQ ID No: 9):
5' LC-Red640 - gcaggtgaagatgatgtcggt - (P) 3'
lysC311-A (SEQ ID No: 10):
5' tcaagatctccatcgcgcggcggccgtcggaacga - fluorescein 3'

The primers shown are synthesized for the PCR by MWG Biotech and oligonucleotides shown for the hybridization are synthesized by TIB MOLBIOL (Berlin, Germany).

A clone which contains the base thymine at position 932 of the coding regions of the two lysC copies and thus has two lysC^{FBR} alleles lysC T311I was identified in this manner.

The strain was called C. glutamicum DSM13992lysC^{FBR} : : lysC^{FBR}.

The strain was deposited as C. glutamicum DSM13992lysC^{FBR}: : lysC^{FBR} on 5th June 2002 under number DSM15036 at the Deutsche Sammlung für Mikroorganismen und Zellkulturen (DSMZ, Braunschweig, Germany) in accordance with the Budapest Treaty.

### Example 2

Generation of a tandem duplication of the lysE gene in the chromosome of Corynebacterium glutamicum

### 2.1. Construction of the tandem vector pK18mobsacB2xlysESma1/1

Plasmid DNA was isolated from the Escherichia coli strain DSM12871 (EP-A-1067193), which carries the plasmid pEC7lysE.

The plasmid contains the lysE gene which codes for lysine export. A pure culture of this strain was deposited on 10th June 1999 at the Deutsche Sammlung für Mikroorganismen und Zellkulturen (DSMZ, Braunschweig, Germany) in accordance with the Budapest Treaty.

The plasmid pEC71lysE is cleaved with the restriction enzyme BamHI (Amersham-Pharmacia, Freiburg, Germany), after separation in an agarose gel (0.8%) the lysE fragment of approx. 1.1 kb is isolated from the agarose gel with the aid of the QIAquick Gel Extraction Kit (Qiagen, Hilden, Germany), and the overhanging ends are completed with Klenow polymerase (Boehringer Mannheim) and employed for ligation with the mobilizable cloning vector pK18mobsacB described by Schäfer et al., Gene, 14, 69-73 (1994). This is cleaved beforehand with the restriction enzyme SmaI and dephosphorylated with alkaline phosphatase (Alkaline Phosphatase, Boehringer Mannheim), mixed with the lysE fragment of approx. 1.1 kb and the mixture is treated with T4 DNA Ligase (Amersham-Pharmacia, Freiburg, Germany).

The E. coli strain DH5α (Grant et al.; Proceedings of the National Academy of Sciences USA, 87 (1990) 4645-4649) is then transformed with the ligation batch (Hanahan, In. DNA Cloning. A Practical Approach. Vol. 1, ILR-Press, Cold Spring Harbor, New York, 1989). Selection of plasmid-carrying cells is made by plating out the transformation batch on LB agar (Sambrook et al., Molecular Cloning: A Laboratory Manual. 2nd Ed., Cold Spring Harbor, New York, 1989), which was supplemented with 25 mg/l kanamycin.

Plasmid DNA is isolated from a transformant with the aid of the QIAprep Spin Miniprep Kit from Qiagen and checked by restriction cleavage with the enzymes BamHI and EcoRI and subsequent agarose gel electrophoresis. The plasmid is called pK18mobsacB1xlysESma1.

In a second step, the plasmid pEC7lysE is in turn cleaved with the restriction enzyme BamHI (Amersham-Pharmacia, Freiburg, Germany), after separation in an agarose gel (0.8%) the lysE fragment of approx. 1.1 kb was isolated from the agarose gel with the aid of the QIAquick Gel Extraction Kit (Qiagen, Hilden, Germany) and employed for ligation with the vector pK18mobsacB1xlysESma1 described in this Example. This is cleaved beforehand with the restriction enzyme BamHI and dephosphorylated with alkaline phosphatase (Alkaline Phosphatase, Boehringer Mannheim), mixed with the lysE fragment of approx. 1.1 kb and the mixture is treated with T4 DNA Ligase (Amersham-Pharmacia, Freiburg, Germany).

The E. coli strain DH5α (Grant et al.; Proceedings of the National Academy of Sciences USA, 87 (1990) 4645-4649) is then transformed with the ligation batch (Hanahan, In. DNA Cloning. A Practical Approach. Vol. 1, ILR-Press, Cold Spring Harbor, New York, 1989). Selection of plasmid-carrying cells is made by plating out the transformation batch on LB agar (Sambrook et al., Molecular Cloning: A Laboratory Manual. 2nd Ed., Cold Spring Harbor, New York, 1989), which was supplemented with 25 mg/l kanamycin.

Plasmid DNA is isolated from a transformant with the aid of the QIAprep Spin Miniprep Kit from Qiagen and checked by restriction cleavage with the enzymes EcoRI and SalI or ScaI and subsequent agarose gel electrophoresis. The plasmid is called pK18mobsacB2xlysESma1/1. A map of the plasmid is shown in Figure 2.

### 2.2. Generation of a tandem duplication of the lysE gene in C. glutamicum strain ATCC21513_17

The vector pK18mobsacB2xlysESma1/1 mentioned in Example 2.1 is transferred by a modified protocol of Schäfer et al. (1990 Journal of Microbiology 172: 1663-1666) into the C. glutamicum strain ATCC21513_17.

The Corynebacterium glutamicum strain ATCC21513_17 was produced by multiple, non-directed mutagenesis, selection and mutant selection from C. glutamicum ATCC21513. The strain is resistant to the lysine analogue S-(2-aminoethyl)-L-cysteine and both leucine- and homoserine-prototrophic.

The vector cannot replicate independently in ATCC21513_17 and is retained in the cell only if it has integrated into the chromosome.

Selection of clones with integrated pK18mobsacB2xlysESma1/1 is carried out by plating out the conjugation batch on LB agar (Sambrook et al., Molecular Cloning: A Laboratory Manual. 2nd Ed., Cold Spring Harbor, New York, 1989), which was supplemented with 15 mg/l kanamycin and 50 mg/l nalidixic acid. Clones which have grown on are plated out on LB agar plates with 25 mg/l kanamycin and incubated for 16 hours at 33°C. To achieve excision of the plasmid with only one copy of the lysE gene, the clones are cultured on LB agar with 10% sucrose, after incubation for 16 hours in LB liquid medium. The plasmid pK18mobsacB contains a copy of the sacB gene, which converts sucrose into levan sucrase, which is toxic to C. glutamicum.

Only those clones in which the pK18mobsacB2xlysESma1/1 integrated has been excised again therefore grow on LB agar with sucrose. Approximately 40 to 50 colonies are tested for the phenotype "growth in the presence of sucrose" and "non-growth in the presence of kanamycin". During the excision, either two copies of the lysE gene or only one can be excised together with the plasmid.

To demonstrate that two copies of lysE have remained in the chromosome, approximately 20 colonies which show the phenotype "growth in the presence of sucrose" and "non-growth in the presence of kanamycin" are investigated with the aid of the polymerase chain reaction by the standard PCR method of Innis et al. (PCR Protocols. A Guide to Methods and Applications, 1990, Academic Press). A DNA fragment which carries the lysE gene and surrounding regions is amplified here from the chromosomal DNA of the colonies. The following primer oligonucleotides are chosen for the PCR.
lysEK-1 (SEQ ID NO: 11):
5' TGC TTG CAC AAG GAC TTC AC 3'
lysEK-2 (SEQ ID NO: 12):
5' TAT GGT CCG CAA GCT CAA TG 3'

The primers allow amplification of a DNA fragment approx. 1.2 kb in size in control clones with the original lysE locus. In clones with a second copy of the lysC gene in the chromosome at the lysE locus, DNA fragments with a size of approx. 2.3 kb are amplified.

The amplified DNA fragments are identified by means of electrophoresis in a 0.8% agarose gel. On the basis of the amplified fragment length, a distinction was made between clones with one chromosomal lysE gene copy and clones with two chromosomal lysE gene copies. It could thus be demonstrated that the strain ATCC21513_17 carries two complete copies of the lysE gene on the chromosome.

The strain was called C. glutamicum ATCC21513_17lysE::lysE.

The strain was deposited as C. glutamicum ATCC21513_17lysE: : lyse on 5th June 2002 under number DSM15037 at the Deutsche Sammlung für Mikroorganismen und Zellkulturen (DSMZ, Braunschweig, Germany) in accordance with the Budapest Treaty.

### Example 3

Generation of a tandem duplication of the zwa1 gene in the chromosome of Corynebacterium glutamicum

### 3.1. Construction of the tandem vector pK18mobsacBzwalzwa1

Plasmid DNA was isolated from the Escherichia coli strain DSM13115 (EP-A-1111062), which carries the plasmid pCR2.1zwalexp.

The plasmid contains the zwa1 gene which codes for cell growth factor 1. A pure culture of this strain was deposited on 19th October 1999 at the Deutsche Sammlung für Mikroorganismen und Zellkulturen (DSMZ, Braunschweig, Germany) in accordance with the Budapest Treaty.

The plasmid pCR2. 1zwa1exp is cleaved with the restriction enzyme EcoRI (Amersham-Pharmacia, Freiburg, Germany), and after separation in an agarose gel (0.8%) the zwa1 fragment of 1 kb is isolated from the agarose gel with the aid of the QIAquick Gel Extraction Kit (Qiagen, Hilden, Germany) and employed for ligation with the mobilizable cloning vector pK18mobsacB described by Schäfer et al., Gene, 14, 69-73 (1994). This is cleaved beforehand with the restriction enzyme EcoRI and dephosphorylated with alkaline phosphatase (Alkaline Phosphatase, Boehringer Mannheim), mixed with the zwa1 fragment of 1 kb and the mixture is treated with T4 DNA Ligase (Amersham-Pharmacia, Freiburg, Germany).

The E. coli strain DH5α (Grant et al.; Proceedings of the National Academy of Sciences USA, 87 (1990) 4645-4649) is then transformed with the ligation batch (Hanahan, In. DNA Cloning. A Practical Approach. Vol. 1, ILR-Press, Cold Spring Harbor, New York, 1989). Selection of plasmid-carrying cells is made by plating out the transformation batch on LB agar (Sambrook et al., Molecular Cloning: A Laboratory Manual. 2nd Ed., Cold Spring Harbor, New York, 1989), which was supplemented with 25 mg/l kanamycin.

Plasmid DNA is isolated from a transformant with the aid of the QIAprep Spin Miniprep Kit from Qiagen and checked by restriction cleavage with the enzyme NheI and subsequent agarose gel electrophoresis. Checking of the plasmid showed that two zwa1 fragments were cloned simultaneously and in the desired orientation in the cloning vector pK18mobsac.

The plasmid is called pK18mobsacBzwa1zwa. A map of the plasmid is shown in Figure 3.

### 3.2. Generation of a tandem duplication of the zwa1 gene in C. glutamicum strain ATCC21513_17

The vector pK18mobsacBzwa1zwa1 mentioned in Example 3.1 is transferred by a modified protocol of Schäfer et al. (1990 Journal of Microbiology 172: 1663-1666) into the C. glutamicum strain ATCC21513_17.

The Corynebacterium glutamicum strain ATCC21513_17 was produced by multiple, non-directed mutagenesis, selection and mutant selection from C. glutamicum ATCC21513. The strain is resistant to the lysine analogue S-(2-aminoethyl)-L-cysteine and both leucine- and homoserine-prototrophic.

The vector cannot replicate independently in ATCC21513_17 and is retained in the cell only if it has integrated into the chromosome.

Selection of clones with integrated pK18mobsacBzwa1zwa1 is carried out by plating out the conjugation batch on LB agar (Sambrook et al., Molecular Cloning: A Laboratory Manual. 2nd Ed., Cold Spring Harbor, New York, 1989), which was supplemented with 15 mg/l kanamycin and 50 mg/l nalidixic acid. Clones which have grown on are plated out on LB agar plates with 25 mg/l kanamycin and incubated for 16 hours at 33°C. To achieve excision of the plasmid with only one copy of the zwa1 gene, the clones are cultured on LB agar with 10% sucrose, after incubation for 16 hours in LB liquid medium. The plasmid pK18mobsacB contains a copy of the sacB gene, which converts sucrose into levan sucrase, which is toxic to C. glutamicum.

Only those clones in which the pK18mobsacBzwa1zwa1 integrated has been excised again therefore grow on LB agar with sucrose. Approximately 40 to 50 colonies are tested for the phenotype "growth in the presence of sucrose" and "non-growth in the presence of kanamycin". During the excision, either two copies of the zwa1 gene or only one can be excised together with the plasmid.

To demonstrate that two copies of zwa1 have remained in the chromosome, approximately 20 colonies which show the phenotype "growth in the presence of sucrose" and "non-growth in the presence of kanamycin" are investigated with the aid of the polymerase chain reaction by the standard PCR method of Innis et al. (PCR Protocols. A Guide to Methods and Applications, 1990, Academic Press). A DNA fragment which carries the zwa1 gene and surrounding regions is amplified here from the chromosomal DNA of the colonies. The following primer oligonucleotides are chosen for the PCR.
zwa1-A2 (SEQ ID NO: 13):
5' CAC TTG TCC TCA CCA CTT TC 3`
zwa1-E1 (SEQ ID NO: 14):
5' TTC TAC TGG GCG TAC TTT CG 3`

The primers allow amplification of a DNA fragment approx. 1.3 kb in size in control clones with the original zwa1 locus. In clones with a second copy of the zwa1 gene in the chromosome at the zwa1 locus, DNA fragments with a size of approx. 2.3 kb are amplified.

The amplified DNA fragments are identified by means of electrophoresis in a 0.8% agarose gel. On the basis of the amplified fragment length, a distinction was made between clones with one chromosomal zwa1 gene copy and clones with two chromosomal zwa1 gene copies. It could thus be demonstrated that the strain ATCC21513_17 carries two complete copies of the zwa1 gene on the chromosome.

The strain was called C. glutamicum ATCC21513_17zwa1: :zwa1. The strain was deposited as C. glutamicum ATCC21513_17 zwa1: :zwa1 on 5th June 2002 under number DSM15038 at the Deutsche Sammlung für Mikroorganismen und Zellkulturen (DSMZ, Braunschweig, Germany) in accordance with the Budapest Treaty.

### Example 4

Preparation of Lysine

The C. glutamicum strains DSM13992lysC^{FBR}: :lysC^{FBR}, ATCC21513_17lysE::lysE and ATCC21513_17zwa1: :zwa1 obtained in Examples 1 to 3 are cultured in a nutrient medium suitable for the production of lysine and the lysine content in the culture supernatant was determined.

For this, the strains are first incubated on an agar plate for 24 hours at 33°C. Starting from this agar plate culture, a preculture is seeded (10 ml medium in a 100 ml conical flask). The medium MM is used as the medium for the preculture. The preculture is incubated for 24 hours at 33°C at 240 rpm on a shaking machine. A main culture is seeded from this preculture such that the initial OD (660 nm) of the main culture is 0.1 OD. The Medium MM is also used for the main culture.

### Medium MM

| | |
|---|---|
| CSL | 5 g/l |
| MOPS | 20 g/l |
| Glucose (autoclaved separately) | 50 g/l |

### Salts:

| | |
|---|---|
| (NH₄)₂SO₄ | 25 g/l |
| KH₂PO₄ | 0.1 g/l |
| MgSO₄ * 7 H₂O | 1 . 0 g/l |
| CaCl₂ * 2 H₂O | 10 mg/l |
| FeSO₄ * 7 H₂O | 10 mg/l |
| MnSO₄ * H₂O | 5.0 mg/l |
| Biotin (sterile-filtered) | 0.3 mg/l |
| Thiamine * HCl (sterile-filtered) | 0.2 mg/l |
| CaCO₃ | 25 g/l |

The CSL (corn steep liquor), MOPS (morpholinopropanesulfonic acid) and the salt solution are brought to pH 7 with aqueous ammonia and autoclaved. The sterile substrate and vitamin solutions, as well as the CaCO₃ autoclaved in the dry state, are then added.

Culturing is carried out in a 10 ml volume in a 100 ml conical flask with baffles. Culturing is carried out at 33°C and 80% atmospheric humidity.

After 48 hours, the OD is determined at a measurement wavelength of 660 nm with a Biomek 1000 (Beckmann Instruments GmbH, Munich). The amount of lysine formed is determined with an amino acid analyzer from Eppendorf-BioTronik (Hamburg, Germany) by ion exchange chromatography and post-column derivation with ninhydrin detection.

The result of the experiment is shown in Table 10.

**Table 10**

| Strain | OD (660 nm) | Lysine HCl g/l |
|---|---|---|
| DSM13992 | 12.8 | 18.9 |
| DSM13992lysC^{FBR} : : lysC^{FBR} | 12.0 | 21.6 |
| ATCC21513_17 | 10.4 | 14.0 |
| ATCC21513-17lysE::lysE | 10.0 | 14.3 |
| ATCC21513_17zwa1::zwa1 | 9.9 | 14.6 |

### Brief Description of the Figures:

The base pair numbers stated are approximate values obtained in the context of reproducibility of measurements.

Figure 1: Map of the plasmid pK18mobsacB2xlysCSma2/1.

The abbreviations and designations used have the following meaning:
- KmR:: Kanamycin resistance gene
- HindIII:: Cleavage site of the restriction enzyme HindIII
- BamHI:: Cleavage site of the restriction enzyme BamHI
- lysC:: lysC^{FBR} allele lysC T311I
- sacB:: sacB gene
- RP4mob:: mob region with the replication origin for the transfer (oriT)
- oriV:: Replication origin V

Figure 2: Map of the plasmid pK18mobsacB2xlysESma1/1.

The abbreviations and designations used have the following meaning:
- KanR:: Kanamycin resistance gene
- SalI:: Cleavage site of the restriction enzyme SalI
- BamHI:: Cleavage site of the restriction enzyme BamHI
- EcoRI:: Cleavage site of the restriction enzyme EcoRI
- ScaI:: Cleavage site of the restriction enzyme ScaI
- lysE:: lysE gene
- sacB:: sacB gene
- RP4mob:: mob region with the replication origin for the transfer (oriT)
- oriV:: Replication origin V

Figure 3: Map of the plasmid pK18mobsacBzwa1zwa1.

The abbreviations and designations used have the following meaning:
- KanR:: Kanamycin resistance gene
- EcoRI:: Cleavage site of the restriction enzyme EcoRI
- NheI:: Cleavage site of the restriction enzyme NheI
- zwa1:: zwa1 gene
- sacB:: sacB gene
- RP4mob:: mob region with the replication origin for the transfer (oriT)
- oriV:: Replication origin V

## Claims

1. Coryneform bacteria with high stability which produce an L-amino acid chosen from the group consisting of L-lysine, L-methionine, L-threonine and L-valine wherein these bacteria comprise at least two copies of a gene whereby said copies are present at the natural site in said bacteria in tandem arrangement and belong to the genes required for the production of said L-amino acids and wherein said genes are chosen from group constisting of:
a) for L-lysine production:
one or more of the genes chosen from the group consisting of accBC, accDA, cstA, cysD, cysE, cysH, cysK, cysN, cysQ, dapA, dapB, dapC, dapD, dapE, dapF, ddh, dps, eno, gap, gap2, gdh, gnd, lysC, lysC^{FBR}, lysE, msiK, opcA, oxyR, ppc, ppc^{FBR}, pgk, pknA, pknB, pknD, pknG, ppsA, ptsH, ptsI, ptsM, pyc, pyc P458S, sigC, sigD, sigE, sigH, sigm, tal, thyA, tkt, tpi, zwa1, zwf and zwf A213T,
b) for L-methionine production:
one or more of the genes chosen from the group consisting of accBC, accDA, aecD, cstA, cysD, cysE, cysH, cysK, cysN, cysQ, dps, eno, fda, gap, gap2, gdh, gnd, glyA, hom, hom^{FBR}, lysC, lysC^{FBR}, metA, metB, metE, metH, metY, msiK, opcA, oxyR, ppc, ppc^{FBR}, pgk, pknA, pknB, pknD, pknG, ppsA, ptsH, ptsI, ptsM, pyc, pyc P458S, sigC, sigD, sigE, sigH, sigM, tal, thyA, tkt, tpi, zwa1, zwf and zwf A213T,
c) for L-threonine production:
one or more of the genes or chosen from the group consisting of accBC, accDA, cstA, cysD, cysE, cysH, cysI, cysN, cysQ, dps, eno, fda, gap, gap2, gdh, gnd, hom, hom^{FBR}, lysC, lysC^{FBR} , msiK, opcA, oxyR, ppc, ppc^{FBR}, pgk, pknA, pknB, pknD, pknG, ppsA, ptsH, ptsI, ptsM, pyc, pyc P458S, sigC, sigD, sigE, sigH, sigM, tal, thyA, tkt, tpi, thrB, thrC, thrE, zwa1, zwf and zwf A213T,
d) for L-valine production:
one or more of the genes chosen from the group consisting of brnE, brnF, brnEF, cstA, cysD, dps, eno, fda, gap, gap2, gdh, ilvB, ilvN, ilvBN, ilvC, ilvD, ilvE msiK, pgk, ptsH, ptsI, ptsM, sigC, sigD, sigE, sigH, sigM, tpi and zwa1,
whereby said bacteria are obtained by transformation using vectors which do not replicate in coryneform bacteria and which carry two copies of said genes in tandem arrangement, and whereby 0 to 24 nucleotides of residues of sequences of the vector used remain on the flanks of said genes.

2. Coryneform bacteria according to claim 1, which have at least a third copy of said gene at a further gene site, whereby 0 to 24 nucleotides of residues of the vector used remain on the flanks of said third copy.

3. Coryneform bacteria according to claims 1 or 2, whereby said bacteria are free from a nucleotide sequence at said sites which provides antibiotic resistance .

4. Coryneform bacteria according to one or more of claims 1 to 3, which produce L-lysine, comprising at least two copies of a gene chosen from the group consisting of lysC^{FBR} , lysE and zwal.

5. Coryneform bacteria according to one or more of claims 1 to 4 which produce L-lysine, comprising at least two copies of a lysE gene.

6. Coryneform bacteria according to one or more of claims 1 to 4 which produce L-lysine, comprising at least two copies of a zwa1 gene.

7. Coryneform bacteria according to one or more of claims 1 to 4 which produce L-lysine, wherein a lysC^{FBR} allele is duplicated which codes for a feed back resistant form of aspartate kinase.

8. Coryneform bacteria according to claim 7, wherein a lysC^{FBR} allele is duplicated which codes for a feed back resistant aspartate kinase which contains one or more amino acid exchanges in the sequence according to SEQ ID NO:2, chosen from the group consisting of A279V, S301F, T308I, S301Y, G345D, R320G, T311I and S381F.

9. Coryneform bacteria according to claim 7, wherein said feed back resistant aspartate kinase has the amino acid sequence according to SEQ ID NO:4.

10. Coryneform bacteria according to claim 7, wherein said lysC^{FBR} allele has the nucleotide sequence according to SEQ ID NO:3.

11. Coryneform bacteria according to claims 1, 9 and 10, comprising a copy of the lysC gene and a copy of the lysC^{FBR} allele, which has the nucleotide sequence according to SEQ ID NO: 3 in tandem arrangement.

12. Coryneform bacteria according to one or more of claims 1 to 10, wherein the coryneform bacteria belong to the genus Corynebacterium.

13. Coryneform bacteria according to claim 12, wherein said bacteria belong to the species Corynebacterium glutamicum.

14. Process for the preparation of an L-amino acid, chosen from the group consisting of L-lysine, L-methionine, L-threonine and L-valine comprising:
a) fermentation of coryneform bacteria according to claims 1 to 13.

15. Process according to claim 14, comprising:
a) concentration of said L-amino acids in the fermentation broth and/or the cells of the bacteria, and
b) isolation of said L-amino acids from the fermentation broth.

16. Process according to claim 15, comprising:
a) isolation of said L-amino acids from the fermentation broth with constituents from the fermentation broth and/or the biomass to the extent of > 0 to 100 %.

17. The plasmid pK18mobsacB2xlysCSma2/1, deposited in the form of a pure culture of the strain E. coli under number DSM14244.

18. The Corynebacterium glutamicum strain DSM139921lysC^{FBR}::lysC^{FBR} deposited in the form of a pure culture under number DSM15036.

19. The Corynebacterium glutamicum strain ATCC21513_17lysE::lysE deposited in the form of a pure culture under DSM15037.

20. The Corynebacterium glutamicum strain ATCC21513_17zwa1::zwa1 deposited in the form of a pure culture under number DSM15038.

## Patentansprüche

1. Coryneforme Bakterien mit hoher Stabilität, die eine L-Aminosäure, ausgewählt aus der Gruppe, bestehend aus L-Lysin, L-Methionin, L-Threonin, und L-Valin, produzieren, wobei diese Bakterien mindestens zwei Kopien eines Gens umfassen, wobei die Kopien an der natürlichen Stelle in den Bakterien in einer Tandemanordnung zugegen sind, und zu den Genen gehören, die zur Produktion von L-Aminosäuren erforderlich sind, und wobei die Gene ausgewählt sind aus der Gruppe, bestehend aus:
a) für die L-Lysin-Produktion:
einem oder mehreren der Gene, ausgewählt aus der Gruppe, bestehend aus:
accBC, accDA, cstA, cysD, cysE, cysH, cysK, cysN, cysQ, dapA, dapB, dapC, dapD, dapE, dapF, ddh, dps, eno, gap, gap2, gdh, gnd, lysC, lysC^{FBR}, lysE, msiK, opcA, oxyR, ppc, ppc^{FBR}, pgk, pknA, pknB, pknD, pknG, ppsA, ptsH, ptsI, ptsM, pyc, pyc P458S, sigC, sigD, sigE, sigH, sigM, tal, thyA, tkt, tpi, zwa1, zwf und zwf A213T,
b) für die L-Methionin-Produktion:
einem oder mehreren der Gene, ausgewählt aus der Gruppe, bestehend aus:
accBC, accDA, aecD, cstA, cysD,
cysE, cysH, cysK, cysN, cysQ, dps, eno, fda, gap, gap2, gdh, gnd, glyA, hom, hom^{FBR}, lysC, lysC^{FBR}, metA, metB, metE, metH, metY, msiK, opcA, oxyR, ppc, ppc^{FBR}, pgk, pknA, pknB, pknD, pknG, ppsA, ptsH, ptsI, ptsM, pyc, pyc P4585S, sigC, sigD, sigE, sigH, sigM, tal, thyA, tkt, tpi, zwa1, zwf und zwf A213T,
c) für die L-Threonin-Produktion:
einem oder mehreren der Gene, ausgewählt aus der Gruppe, bestehend aus:
accBC, accDA, cstA, cysD, cysE, cysH, cysI, cysN, cysQ, dps, eno, fda, gap, gap2, gdh, gnd, hom, hom^{FBR}, lysC, lysC^{FBR}, msiK, opcA, oxyR, ppa, ppc, ppc^{FBR}, pgk, pknA, pknB, pknD, pknG, ppsA, ptsH, ptsI, ptsM, pyc, pyc P458S, sigC, sigD, sigE, sigH, sigM, tal, thyA, tkt, tpi, thrB, thrC, thrE, zwa1, zwf und zwf A213T,
d) für die L-Valin-Produktion:
einem oder mehrere der Gene, ausgewählt aus der Gruppe, bestehend aus:
brnE, brnF, brnEF, cstA, cysD, dps, eno, fda, gap, gap2, gdh, ilvB, ilvN, ilvBN, ilvC, ilvD, ilvE, msiK, pgk, ptsH, ptsI, ptsM, sigc, sigD, sigE, sigH, sigM, tpi und zwa1,
wobei die Bakterien durch Transformation mit den Vektoren, die nicht in coryneformen Bakterien replizieren, und die die beiden Kopien der Gene in Tandemanordnung tragen, erhalten werden, und wobei 0 bis 24 Nukleotide der Reste der Sequenzen des verwendeten Vektors auf den Flanken der Gene verbleiben.

2. Coryneforme Bakterien nach Anspruch 1, die mindestens eine dritte Kopie des Gens an einer weiteren Genstelle aufweisen, wobei 0 bis 24 Nukleotide der Reste des verwendeten Vektors auf den Flanken der dritten Kopie verbleiben.

3. Coryneforme Bakterien nach den Ansprüchen 1 oder 2, wobei die Bakterien frei von einer Nukleotidsequenz an den Stellen sind, die eine Antibiotika-Resistenz bereitstellen.

4. Coryneforme Bakterien nach einem oder mehreren der Ansprüche 1 bis 3, die L-Lysin produzieren, umfassend mindestens zwei Kopien eines Gens, ausgewählt aus der Gruppe, bestehend aus lysC^{FBR}, lysE and zwa1.

5. Coryneforme Bakterien nach einem oder mehreren der Ansprüche 1 bis 4, die L-Lysin produzieren, umfassend mindestens zwei Kopien eines lysE-Gens.

6. Coryneforme Bakterie nach einem oder mehreren der Ansprüche 1 bis 4, die L-Lysin produzieren, umfassend mindestens zwei Kopien eines zwal-Gens.

7. Coryneforme Bakterien nach einem oder mehreren der Ansprüche 1 bis 4, die L-Lysin produzieren, wobei ein lysC^{FBR}-Allel dupliziert ist, das eine Rückkopplungs-resistente Form von Aspartatkinase codiert.

8. Coryneforme Bakterien nach Anspruch 7, wobei ein lysC^{FBR}-Allel dupliziert ist, das eine Rückkopplungs-resistente Form von Aspartatkinase codiert, die eine oder mehrere Aminosäureaustausche in der Sequenz gemäß SEQ ID NR: 2 enthält, ausgewählt aus der Gruppe, bestehend aus A279V, S301F, T308I, S301Y, G345D, R320G, T311I und S381F.

9. Coryneforme Bakterien nach Anspruch 7, wobei die Rückkopplungs-resistente Aspartatkinase die Aminosäuresequenz gemäß SEQ ID NR: 4 hat.

10. Coryneforme Bakterien nach Anspruch 7, wobei das lysC^{FBR}-Allel die Nukleotidsequenz gemäß SEQ ID NR: 3 hat.

11. Coryneforme Bakterien nach Anspruch 1, 9 und 10, umfassend eine Kopie des lysC-Gens und eine Kopie des lysC^{FBR}-Allels, das die Nukleotidsequenz gemäß SEQ ID NR: 3 hat, in Tandem-Anordnung.

12. Coryneforme Bakterien nach einem oder mehreren der Ansprüche 1 bis 10, wobei die coryneformen Bakterien zur Gattung Corynebacterium gehören.

13. Coryneforme Bakterien nach Anspruch 12, wobei die Bakterien zur Art Corynebacterium glutamicum gehören.

14. Verfahren zur Herstellung einer L-Aminosäure, ausgewählt aus der Gruppe, bestehend aus L-Lysin, L-Methionin, L-Threonin und L-Valin, umfassend:
a) Fermentierung coryneformer Bakterien nach den Ansprüchen 1 bis 13.

15. Verfahren nach Anspruch 14, umfassend:
a) Aufkonzentrieren der L-Aminosäuren in der Fermentationsbrühe und/oder den Bakterienzellen, und
b) Isolation der L-Aminosäuren aus der Fermentationsbrühe.

16. Verfahren nach Anspruch 15, umfassend:
a) Isolieren der L-Aminosäuren aus der Fermentationsbrühe mit Bestandteilen aus der Fermentationsbrühe und/oder der Biomasse in einem Ausmaß von >0 bis 100%.

17. Plasmid pK18mobsacB2xlysCSma2/1, hinterlegt in der Form einer reinen Kultur des Stamms E. coli unter der Nummer DSM14244.

18. Corynebacterium glutamicum Stamm DSM139921lysC^{FBR} ::lysC^{FBR}, hinterlegt in der Form einer reinen Kultur unter der Nummer DSM15036.

19. Corynebacterium glutamicum Stamm ATCC21513_171lysE::lysE, hinterlegt in der Form einer reinen Kultur unter der Nummer DSM15037.

20. Corynebacterium glutamicum Stamm ATCC21513_17zwa1::zwa1, hinterlegt in der Form einer reinen Kultur unter der Nummer DSM15038.

## Revendications

1. Bactéries corynéforme à haute stabilité qui produisent un acide aminé L choisi dans le groupe constitué de la L-lysine, la L-méthionine, la L-thréonine et la L-valine **caractérisées en ce que** ces bactéries comprennent au moins deux copies d'un gène de telle manière que lesdites copies soient présentes au site naturel dans lesdites bactéries agencées en tandem et appartiennent aux gènes requis pour la production desdits acides aminés L et **caractérisées en ce que** lesdits gènes sont choisis dans le groupe constitué de :
a) pour la production de L-lysine :
un ou plusieurs des gènes choisis dans le groupe constitué de accBC, accDA, cstA, cysD, cysE, cysH, cysK, cysN, cysQ, dapA, dapB, dapC, dapD, dapE, dapF, ddh, dps, eno, gap, gap2, gdh, gnd, lysC, lysC^{FBR}, lysE, msiK, opcA, oxyR, ppc, ppc^{FBR}, pgk, pknA, pknB, pknD, pknG, ppsA, ptsH, ptsI, ptsM, pyc, pyc P458S, sigC, sigD, sigE, sigH, sigM, tal, thyA, tkt, tpi, zwa1, zwf et zwf A213T,
b) pour la production de L-méthionine :
un ou plusieurs des gènes choisis dans le groupe constitué de accBC, accDA, aecD, cstA, cysE, cysE, cysH, cysK, cySN, cysQ, dps, eno, fda, gap, gap2, gdh, gnd, glyA, hom, hom^{FBR}, lysC, lysC^{FSR}, metA, metB, metE, metH, metY, msiK, opcA, oxyR, ppc, ppc^{FBR}, pgk, pknA, pknB, pknD, pknG, ppsA, ptsH, ptsI, ptsM, pyc, pyc P458S, sigC, sigD, sigE, sigH, sigM, tal, thyA, tkt, tpi, zwa1, zwf et zwf A213T,
c) pour la production de L-thréonine :
un ou plusieurs des gènes choisis dans le groupe constitué de accBC, accDA, cstA, cysD, cysE, cysH, cysI, cysN, cysQ, dps, eno, fda, gap, gap2, gdh, gnd, hom, hom^{FBR}, lysC, lysC^{FBR}, msiK, opcA, oxyR, ppc, ppc^{FBR}, pgk, pknA, pknB, pknD, pknG, ppsA, ptsH, ptsI, ptsM, pyc, pyc P458S, sigC, sigD, sigE, sigH, sigM, tal, thyA, tkt, tpi, thrB, thrC, thrE, zwa1, zwf et zwf A213T,
d) pour la production de L-valine :
un ou plusieurs des gènes choisis dans le groupe constitué de brnE, brnF, brnEF, cstA, cysD, dps, eno, fda, gap, gap2, gdh, ilvB, ilvN, ilvBN, ilvC, ilvD, ilvE msiK, pgk, ptsH, ptsI, ptsM, sigC, sigD, sigE, sigH, sigM, tpi et zwa1,
de telle manière que lesdites bactéries soient obtenues par transformation en utilisant des vecteurs qui ne se répliquent pas dans des bactéries corynéformes et qui portent deux copies desdits gènes agencés en tandem, et de telle manière qu'il reste de 0 à 24 nucléotides de résidus de séquences du vecteur utilisé sur les flancs desdits gènes.

2. Bactéries corynéformes selon la revendication 1, qui ont au moins une troisième copie dudit gène à un site de gène supplémentaire, de telle manière qu'il reste de 0 à 24 nucléotides de résidus du vecteur utilisé sur les flancs de ladite troisième copie.

3. Bactéries corynéformes selon la revendication 1 ou 2, **caractérisées en ce que** lesdites bactéries ne comportent pas une séquence nucléotidique auxdits sites qui confère une résistance aux antibiotiques.

4. Bactéries corynéformes selon une ou plusieurs des revendications 1 à 3, qui produisent de la L-lysine, comprenant au moins deux copies d'un gène choisi dans le groupe constitué de lysC^{FBR}, lysE et zwa1.

5. Bactéries corynéformes selon une ou plusieurs des revendications 1 à 4, qui produisent de la L-lysine, comprenant au moins deux copies d'un gène lysE.

6. Bactéries corynéformes selon une ou plusieurs des revendications 1 à 4, qui produisent de la L-lysine, comprenant au moins deux copies d'un gène zwa1.

7. Bactéries corynéformes selon une ou plusieurs des revendications 1 à 4, qui produisent de la L-lysine, **caractérisées en ce qu'**un allèle lysC^{FBR} est dupliqué qui code pour une forme résistante rétroactive d'aspartate kinase.

8. Bactéries corynéformes selon la revendication 7, **caractérisées en ce qu'**un allèle lysC^{FBR} est dupliqué qui code pour une aspartate kinase résistante rétroactive qui contient une ou plusieurs substitutions d'acide aminé dans la séquence conformément à SEQ ID N° 2, choisies dans le groupe constitué de A279V, S301F, T308I, S301Y, G345D, R320G, T311I et S381F.

9. Bactéries corynéformes selon la revendication 7, **caractérisées en ce que** ladite aspactate kinase résistante rétroactive a la séquence d'acide aminé selon SEQ ID N° 4.

10. Bactéries corynéformes selon la revendication 7, **caractérisées en ce que** ledit allèle lysC^{FBR} a la séquence nucléotidique selon SEQ ID N° 3.

11. Bactéries corynéformes selon les revendications 1, 9 et 10, comprenant une copie du gène lysC et une copie de l'allèle lysC^{FBR}, qui a la séquence nucléotidique selon SEQ ID N° 3 agencée en tandem.

12. Bactéries corynéformes selon une ou plusieurs des revendications 1 à 10, **caractérisées en ce que** les bactéries corynéformes appartiennent au genre Corynebacterium.

13. Bactéries corynéformes selon la revendication 12, **caractérisées en ce que** lesdites bactéries appartiennent à l'espèce Corynebacterium glutamicum.

14. Procédé pour la préparation d'un acide aminé L, choisi dans le groupe constitué de la L-lysine, la L-méthionine, la L-thréonine et la L-valine comprenant :
a) la fermentation de bactéries corynéformes selon les revendications 1 à 13.

15. Procédé selon la revendication 14, comprenant :
a) la concentration desdits acides aminés L dans le milieu de fermentation et/ou les cellules des bactéries, et
b) l'isolement desdits acides aminés L à partir du milieu de fermentation.

16. Procédé selon la revendication 15, comprenant :
a) l'isolement desdits acides aminés L à partir du milieu de fermentation avec des constituants du milieu de fermentation et/ou la biomasse à un taux de > 0 à 100 %.

17. Plasmide pK18mobsacB2xlysCSma2/1, déposé sous la forme d'une culture pure de la souche E. coli numéro DSM14244.

18. Souche de Corynebacterium glutamicum DSM139921lysC^{FBR}: : lysC^{FBR} déposée sous la forme d'une culture pure sous le numéro DSM15036.

19. Souche de Corynebacterium glutamicum ATCC21513_17lysE::lysE déposée sous la forme d'une culture pure sous le numéro DSM15037.

20. Souche de Corynebacterium glutamicum ATCC21513_17zwa1: :zwa1 déposée sous la forme d'une culture pure sous le numéro DSM15038.


### SEQUENCE LISTING

<110> Degussa AG
<120> Coryneform bacteria which produce chemical compounds II
<130> 010303 BT
<160> 14
<170> PatentIn version 3.1
<210> 1
   <211> 1263
   <212> DNA
   <213> Corynebacterium glutamicum
<220>
   <221> CDS
   <222> (1)..(1263)
   <223> lysC wild-type gene
<400> 1
   gtg gcc ctg gtc gta cag aaa tat ggc ggt tcc tcg ctt gag agt gcg      48
   Met Ala Leu Val Val Gln Lys Tyr Gly Gly Ser Ser Leu Glu Ser Ala
   1               5                   10                  15
   gaa cgc att aga aac gtc gct gaa cgg atc gtt gcc acc aag aag gct      96
   Glu Arg Ile Arg Asn Val Ala Glu Arg Ile Val Ala Thr Lys Lys Ala
               20                  25                  30
   gga aat gat gtc gtg gtt gtc tgc tcc gca atg gga gac acc acg gat     144
   Gly Asn Asp Val Val Val Val Cys Ser Ala Met Gly Asp Thr Thr Asp
           35                  40                  45
   gaa ctt cta gaa ctt gca gcg gca gtg aat ccc gtt ccg cca gct cgt     192
   Glu Leu Leu Glu Leu Ala Ala Ala Val Asn Pro Val Pro Pro Ala Arg
       50                  55                  60
   gaa atg gat atg ctc ctg act gct ggt gag cgt att tct aac gct ctc     240
   Glu Met Asp Met Leu Leu Thr Ala Gly Glu Arg Ile Ser Asn Ala Leu
   65                  70                  75                  80
   gtc gcc atg gct att gag tcc ctt ggc gca gaa gcc caa tct ttc acg     288
   Val Ala Met Ala Ile Glu Ser Leu Gly Ala Glu Ala Gln Ser Phe Thr
                   85                  90                  95
   ggc tct cag gct ggt gtg ctc acc acc gag cgc cac gga aac gca cgc     336
   Gly Ser Gln Ala Gly Val Leu Thr Thr Glu Arg His Gly Asn Ala Arg
               100                 105                 110
   att gtt gat gtc act cca ggt cgt gtg cgt gaa gca ctc gat gag ggc     384
   Ile Val Asp Val Thr Pro Gly Arg Val Arg Glu Ala Leu Asp Glu Gly
           115                 120                 125
   aag atc tgc att gtt gct ggt ttc cag ggt gtt aat aaa gaa acc cgc     432
   Lys Ile Cys Ile Val Ala Gly Phe Gln Gly Val Asn Lys Glu Thr Arg
       130                 135                 140
   gat gtc acc acg ttg ggt cgt ggt ggt tct gac acc act gca gtt gcg      480
   Asp Val Thr Thr Leu Gly Arg Gly Gly Ser Asp Thr Thr Ala Val Ala
   145                 150                 155                 160
   ttg gca gct gct ttg aac gct gat gtg tgt gag att tac tcg gac gtt      528
   Leu Ala Ala Ala Leu Asn Ala Asp Val Cys Glu Ile Tyr Ser Asp Val
                   165                 170                 175
   gac ggt gtg tat acc gct gac ccg cgc atc gtt cct aat gca cag aag      576
   Asp Gly Val Tyr Thr Ala Asp Pro Arg Ile Val Pro Asn Ala Gln Lys
               180                 185                 190
   ctg gaa aag ctc agc ttc gaa gaa atg ctg gaa ctt gct gct gtt ggc      624
   Leu Glu Lys Leu Ser Phe Glu Glu Met Leu Glu Leu Ala Ala Val Gly
           195                 200                 205
   tcc aag att ttg gtg ctg cgc agt gtt gaa tac gct cgt gca ttc aat      672
   Ser Lys Ile Leu Val Leu Arg Ser Val Glu Tyr Ala Arg Ala Phe Asn
       210                 215                 220
   gtg cca ctt cgc gta cgc tcg tct tat agt aat gat ccc ggc act ttg      720
   Val Pro Leu Arg Val Arg Ser Ser Tyr Ser Asn Asp Pro Gly Thr Leu
   225                 230                 235                 240
   att gcc ggc tct atg gag gat att cct gtg gaa gaa gca gtc ctt acc      768
   Ile Ala Gly Ser Met Glu Asp Ile Pro Val Glu Glu Ala Val Leu Thr
                   245                 250                 255
   ggt gtc gca acc gac aag tcc gaa gcc aaa gta acc gtt ctg ggt att      816
   Gly Val Ala Thr Asp Lys Ser Glu Ala Lys Val Thr Val Leu Gly Ile
               260                 265                 270
   tcc gat aag cca ggc gag gct gcg aag gtt ttc cgt gcg ttg gct gat      864
   Ser Asp Lys Pro Gly Glu Ala Ala Lys Val Phe Arg Ala Leu Ala Asp
           275                 280                 285
   gca gaa atc aac att gac atg gtt ctg cag aac gtc tct tct gta gaa      912
   Ala Glu Ile Asn Ile Asp Met Val Leu Gln Asn Val Ser Ser Val Glu
       290                 295                 300
   gac ggc acc acc gac atc acc ttc acc tgc cct cgt tcc gac ggc cgc      960
   Asp Gly Thr Thr Asp Ile Thr Phe Thr Cys Pro Arg Ser Asp Gly Arg
   305                 310                 315                 320
   cgc gcg atg gag atc ttg aag aag ctt cag gtt cag ggc aac tgg acc     1008
   Arg Ala Met Glu Ile Leu Lys Lys Leu Gln Val Gln Gly Asn Trp Thr
                   325                 330                 335
   aat gtg ctt tac gac gac cag gtc ggc aaa gtc tcc ctc gtg ggt gct     1056
   Asn Val Leu Tyr Asp Asp Gln Val Gly Lys Val Ser Leu Val Gly Ala
               340                 345                 350
   ggc atg aag tct cac cca ggt gtt acc gca gag ttc atg gaa gct ctg     1104
   Gly Met Lys Ser His Pro Gly Val Thr Ala Glu Phe Met Glu Ala Leu
           355                 360                 365
   cgc gat gtc aac gtg aac atc gaa ttg att tcc acc tct gag att cgt     1152
   Arg Asp Val Asn Val Asn Ile Glu Leu Ile Ser Thr Ser Glu Ile Arg
       370                 375                 380
   att tcc gtg ctg atc cgt gaa gat gat ctg gat gct gct gca cgt gca      1200
   Ile Ser Val Leu Ile Arg Glu Asp Asp Leu Asp Ala Ala Ala Arg Ala
   385                 390                 395                 400
   ttg cat gag cag ttc cag ctg ggc ggc gaa gac gaa gcc gtc gtt tat      1248
   Leu His Glu Gln Phe Gln Leu Gly Gly Glu Asp Glu Ala Val Val Tyr
                   405                 410                 415
   gca ggc acc gga cgc                                                   1263
   Ala Gly Thr Gly Arg
               420
<210> 2
   <211> 421
   <212> PRT
   <213> Corynebacterium glutamicum
<400> 2
   Met Ala Leu Val Val Gln Lys Tyr Gly Gly Ser Ser Leu Glu Ser Ala
   1               5                   10                  15
   Glu Arg Ile Arg Asn Val Ala Glu Arg Ile Val Ala Thr Lys Lys Ala
               20                  25                  30
   Gly Asn Asp Val Val Val Val Cys Ser Ala Met Gly Asp Thr Thr Asp
           35                  40                  45
   Glu Leu Leu Glu Leu Ala Ala Ala Val Asn Pro Val Pro Pro Ala Arg
       50                  55                  60
   Glu Met Asp Met Leu Leu Thr Ala Gly Glu Arg Ile Ser Asn Ala Leu
   65                  70                  75                  80
   Val Ala Met Ala Ile Glu Ser Leu Gly Ala Glu Ala Gln Ser Phe Thr
                   85                  90                  95
   Gly Ser Gln Ala Gly Val Leu Thr Thr Glu Arg His Gly Asn Ala Arg
               100                 105                 110
   Ile Val Asp Val Thr Pro Gly Arg Val Arg Glu Ala Leu Asp Glu Gly
           115                 120                 125
   Lys Ile Cys Ile Val Ala Gly Phe Gln Gly Val Asn Lys Glu Thr Arg
       130                 135                 140
   Asp Val Thr Thr Leu Gly Arg Gly Gly Ser Asp Thr Thr Ala Val Ala
   145                 150                 155                 160
   Leu Ala Ala Ala Leu Asn Ala Asp Val Cys Glu Ile Tyr Ser Asp Val
                   165                 170                 175
   Asp Gly Val Tyr Thr Ala Asp Pro Arg Ile Val Pro Asn Ala Gln Lys
               180                 185                 190
   Leu Glu Lys Leu Ser Phe Glu Glu Met Leu Glu Leu Ala Ala Val Gly
           195                 200                 205
   Ser Lys Ile Leu Val Leu Arg Ser Val Glu Tyr Ala Arg Ala Phe Asn
       210                 215                 220
   Val Pro Leu Arg Val Arg Ser Ser Tyr Ser Asn Asp Pro Gly Thr Leu
   225                 230                 235                 240
   Ile Ala Gly Ser Met Glu Asp Ile Pro Val Glu Glu Ala Val Leu Thr
                   245                 250                 255
   Gly Val Ala Thr Asp Lys Ser Glu Ala Lys Val Thr Val Leu Gly Ile
               260                 265                 270
   Ser Asp Lys Pro Gly Glu Ala Ala Lys Val Phe Arg Ala Leu Ala Asp
           275                 280                 285
   Ala Glu Ile Asn Ile Asp Met Val Leu Gln Asn Val Ser Ser Val Glu
       290                 295                 300
   Asp Gly Thr Thr Asp Ile Thr Phe Thr Cys Pro Arg Ser Asp Gly Arg
   305                 310                 315                 320
   Arg Ala Met Glu Ile Leu Lys Lys Leu Gln Val Gln Gly Asn Trp Thr
                   325                 330                 335
   Asn Val Leu Tyr Asp Asp Gln Val Gly Lys Val Ser Leu Val Gly Ala
               340                 345                 350
   Gly Met Lys Ser His Pro Gly Val Thr Ala Glu Phe Met Glu Ala Leu
           355                 360                 365
   Arg Asp Val Asn Val Asn Ile Glu Leu Ile Ser Thr Ser Glu Ile Arg
       370                 375                 380
   Ile Ser Val Leu Ile Arg Glu Asp Asp Leu Asp Ala Ala Ala Arg Ala
   385                 390                 395                 400
   Leu His Glu Gln Phe Gln Leu Gly Gly Glu Asp Glu Ala Val Val Tyr
                   405                 410                 415
   Ala Gly Thr Gly Arg
               420
<210> 3
   <211> 1263
   <212> DNA
   <213> Corynebacterium glutamicum
<220>
   <221> CDS
   <222> (1)..(1263)
   <223> lysC-fbr allele lysC T311I
<400> 3
   gtg gcc ctg gtc gta cag aaa tat ggc ggt tcc tcg ctt gag agt gcg       48
   Met Ala Leu Val Val Gln Lys Tyr Gly Gly Ser Ser Leu Glu Ser Ala
   1               5                   10                  15
   gaa cgc att aga aac gtc gct gaa cgg atc gtt gcc acc aag aag gct       96
   Glu Arg Ile Arg Asn Val Ala Glu Arg Ile Val Ala Thr Lys Lys Ala
               20                  25                  30
   gga aat gat gtc gtg gtt gtc tgc tcc gca atg gga gac acc acg gat      144
   Gly Asn Asp Val Val Val Val Cys Ser Ala Met Gly Asp Thr Thr Asp
           35                  40                  45
   gaa ctt cta gaa ctt gca gcg gca gtg aat ccc gtt ccg cca gct cgt 192
   Glu Leu Leu Glu Leu Ala Ala Ala Val Asn Pro Val Pro Pro Ala Arg
   50 55 60
   gaa atg gat atg ctc ctg act gct ggt gag cgt att tct aac gct ctc 240
   Glu Met Asp Met Leu Leu Thr Ala Gly Glu Arg Ile Ser Asn Ala Leu
   65 70 75 80
   gtc gcc atg gct att gag tcc ctt ggc gca gaa gcc caa tct ttc acg 288
   Val Ala Met Ala Ile Glu Ser Leu Gly Ala Glu Ala Gln Ser Phe Thr
   85 90 95
   ggc tct cag gct ggt gtg ctc acc acc gag cgc cac gga aac gca cgc 336
   Gly Ser Gln Ala Gly Val Leu Thr Thr Glu Arg His Gly Asn Ala Arg
   100 105 110
   att gtt gat gtc act cca ggt cgt gtg cgt gaa gca ctc gat gag ggc 384
   Ile Val Asp Val Thr Pro Gly Arg Val Arg Glu Ala Leu Asp Glu Gly
   115 120 125
   aag atc tgc att gtt gct ggt ttc cag ggt gtt aat aaa gaa acc cgc 432
   Lys Ile Cys Ile Val Ala Gly Phe Gln Gly Val Asn Lys Glu Thr Arg
   130 135 140
   gat gtc acc acg ttg ggt cgt ggt ggt tct gac acc act gca gtt gcg 480
   Asp Val Thr Thr Leu Gly Arg Gly Gly Ser Asp Thr Thr Ala Val Ala
   145 150 155 160
   ttg gca gct gct ttg aac gct gat gtg tgt gag att tac tcg gac gtt 528
   Leu Ala Ala Ala Leu Asn Ala Asp Val Cys Glu Ile Tyr Ser Asp Val
   165 170 175
   gac ggt gtg tat acc gct gac ccg cgc atc gtt cct aat gca cag aag 576
   Asp Gly Val Tyr Thr Ala Asp Pro Arg Ile Val Pro Asn Ala Gln Lys
   180 185 190
   ctg gaa aag ctc agc ttc gaa gaa atg ctg gaa ctt gct gct gtt ggc 624
   Leu Glu Lys Leu Ser Phe Glu Glu Met Leu Glu Leu Ala Ala Val Gly
   195 200 205
   tcc aag att ttg gtg ctg cgc agt gtt gaa tac gct cgt gca ttc aat 672
   Ser Lys Ile Leu Val Leu Arg Ser Val Glu Tyr Ala Arg Ala Phe Asn
   210 215 220
   gtg cca ctt cgc gta cgc tcg tct tat agt aat gat ccc ggc act ttg 720
   Val Pro Leu Arg Val Arg Ser Ser Tyr Ser Asn Asp Pro Gly Thr Leu
   225 230 235 240
   att gcc ggc tct atg gag gat att cct gtg gaa gaa gca gtc ctt acc 768
   Ile Ala Gly Ser Met Glu Asp Ile Pro Val Glu Glu Ala Val Leu Thr
   245 250 255
   ggt gtc gca acc gac aag tcc gaa gcc aaa gta acc gtt ctg ggt att 816
   Gly Val Ala Thr Asp Lys Ser Glu Ala Lys Val Thr Val Leu Gly Ile
   260 265 270
   tcc gat aag cca ggc gag gct gcg aag gtt ttc cgt gcg ttg gct gat 864
   Ser Asp Lys Pro Gly Glu Ala Ala Lys Val Phe Arg Ala Leu Ala Asp
   275 280 285
   gca gaa atc aac att gac atg gtt ctg cag aac gtc tct tct gta gaa        912
   Ala Glu Ile Asn Ile Asp Met Val Leu Gln Asn Val Ser Ser Val Glu
       290                 295                 300
   gac ggc acc acc gac atc atc ttc acc tgc cct cgt tcc gac ggc cgc        960
   Asp Gly Thr Thr Asp Ile Ile Phe Thr Cys Pro Arg Ser Asp Gly Arg
   305                 310                 315                 320
   cgc gcg atg gag atc ttg aag aag ctt cag gtt cag ggc aac tgg acc       1008
   Arg Ala Met Glu Ile Leu Lys Lys Leu Gln Val Gln Gly Asn Trp Thr
                   325                 330                 335
   aat gtg ctt tac gac gac cag gtc ggc aaa gtc tcc ctc gtg ggt gct       1056
   Asn Val Leu Tyr Asp Asp Gln Val Gly Lys Val Ser Leu Val Gly Ala
               340                 345                 350
   ggc atg aag tct cac cca ggt gtt acc gca gag ttc atg gaa gct ctg       1104
   Gly Met Lys Ser His Pro Gly Val Thr Ala Glu Phe Met Glu Ala Leu
           355                 360                 365
   cgc gat gtc aac gtg aac atc gaa ttg att tcc acc tct gag att cgt       1152
   Arg Asp Val Asn Val Asn Ile Glu Leu Ile Ser Thr Ser Glu Ile Arg
       370                 375                 380
   att tcc gtg ctg atc cgt gaa gat gat ctg gat gct gct gca cgt gca       1200
   Ile Ser Val Leu Ile Arg Glu Asp Asp Leu Asp Ala Ala Ala Arg Ala
   385                 390                 395                 400
   ttg cat gag cag ttc cag ctg ggc ggc gaa gac gaa gcc gtc gtt tat       1248
   Leu His Glu Gln Phe Gln Leu Gly Gly Glu Asp Glu Ala Val Val Tyr
                   405                 410                 415
   gca ggc acc gga cgc                                                   1263
   Ala Gly Thr Gly Arg
               420
<210> 4
   <211> 421
   <212> PRT
   <213> Corynebacterium glutamicum
<400> 4
   Met Ala Leu Val Val Gln Lys Tyr Gly Gly Ser Ser Leu Glu Ser Ala
   1               5                   10                  15
   Glu Arg Ile Arg Asn Val Ala Glu Arg Ile Val Ala Thr Lys Lys Ala
               20                  25                  30
   Gly Asn Asp Val Val Val Val Cys Ser Ala Met Gly Asp Thr Thr Asp
           35                  40                  45
   Glu Leu Leu Glu Leu Ala Ala Ala Val Asn Pro Val Pro Pro Ala Arg
       50                  55                  60
   Glu Met Asp Met Leu Leu Thr Ala Gly Glu Arg Ile Ser Asn Ala Leu
   65                  70                  75                  80
   Val Ala Met Ala Ile Glu Ser Leu Gly Ala Glu Ala Gln Ser Phe Thr
                   85                  90                  95
   Gly Ser Gln Ala Gly Val Leu Thr Thr Glu Arg His Gly Asn Ala Arg
   100 105 110
   Ile Val Asp Val Thr Pro Gly Arg Val Arg Glu Ala Leu Asp Glu Gly
   115 120 125
   Lys Ile Cys Ile Val Ala Gly Phe Gln Gly Val Asn Lys Glu Thr Arg
   130 135 140
   Asp Val Thr Thr Leu Gly Arg Gly Gly Ser Asp Thr Thr Ala Val Ala
   145 150 155 160
   Leu Ala Ala Ala Leu Asn Ala Asp Val Cys Glu Ile Tyr Ser Asp Val
   165 170 175
   Asp Gly Val Tyr Thr Ala Asp Pro Arg Ile Val Pro Asn Ala Gln Lys
   180 185 190
   Leu Glu Lys Leu Ser Phe Glu Glu Met Leu Glu Leu Ala Ala Val Gly
   195 200 205
   Ser Lys Ile Leu Val Leu Arg Ser Val Glu Tyr Ala Arg Ala Phe Asn
   210 215 220
   Val Pro Leu Arg Val Arg Ser Ser Tyr Ser Asn Asp Pro Gly Thr Leu
   225 230 235 240
   Ile Ala Gly Ser Met Glu Asp Ile Pro Val Glu Glu Ala Val Leu Thr
   245 250 255
   Gly Val Ala Thr Asp Lys Ser Glu Ala Lys Val Thr Val Leu Gly Ile
   260 265 270
   Ser Asp Lys Pro Gly Glu Ala Ala Lys Val Phe Arg Ala Leu Ala Asp
   275 280 285
   Ala Glu Ile Asn Ile Asp Met Val Leu Gln Asn Val Ser Ser Val Glu
   290 295 300
   Asp Gly Thr Thr Asp Ile Ile Phe Thr Cys Pro Arg Ser Asp Gly Arg
   305 310 315 320
   Arg Ala Met Glu Ile Leu Lys Lys Leu Gln Val Gln Gly Asn Trp Thr
   325 330 335
   Asn Val Leu Tyr Asp Asp Gln Val Gly Lys Val Ser Leu Val Gly Ala
   340 345 350
   Gly Met Lys Ser His Pro Gly Val Thr Ala Glu Phe Met Glu Ala Leu
   355 360 365
   Arg Asp Val Asn Val Asn Ile Glu Leu Ile Ser Thr Ser Glu Ile Arg
   370 375 380
   Ile Ser Val Leu Ile Arg Glu Asp Asp Leu Asp Ala Ala Ala Arg Ala
   385 390 395 400
   Leu His Glu Gln Phe Gln Leu Gly Gly Glu Asp Glu Ala Val Val Tyr
   405 410 415
   Ala Gly Thr Gly Arg
   420
<210> 5
   <211> 20
   <212> DNA
   <213> Corynebacterium glutamicum
<220>
   <221> misc_feature
   <222> (1)..(20)
   <223> Primer lysCK1
<400> 5
   tcggtgtcat cagagcattg 20
<210> 6
   <211> 20
   <212> DNA
   <213> Corynebacterium glutamicum
<220>
   <221> misc_feature
   <222> (1)..(20)
   <223> Primer lysCK2
<400> 6
   tcggttgcct gagtaatgtc 20
<210> 7
   <211> 20
   <212> DNA
   <213> Corynebacterium glutamicum
<220>
   <221> misc_feature
   <222> (1)..(20)
   <223> LC-lysC1-fbr
<400> 7
   aaccgttctg ggtatttccg 20
<210> 8
   <211> 20
   <212> DNA
   <213> Corynebacterium glutamicum
<220>
   <221> misc_feature
   <222> (1)..(20)
   <223> LC-lysC2-fbr
<400> 8
   tccatgaact ctgcggtaac 20
<210> 9
   <211> 21
   <212> DNA
   <213> Corynebacterium glutamicum
<220>
   <221> misc_feature
   <222> (1)..(21)
   <223> oligonucleotide lysC311-C
<400> 9
   gcaggtgaag atgatgtcgg t 21
<210> 10
   <211> 35
   <212> DNA
   <213> Corynebacterium.glutamicum
<220>
   <221> misc_feature
   <222> (1)..(35)
   <223> Oligonukleotid lysC311-A
<400> 10
   tcaagatctc catcgcgcgg cggccgtcgg aacga 35
<210> 11
   <211> 20
   <212> DNA
   <213> Corynebacterium glutamicum
<220>
   <221> misc_feature
   <222> (1)..(20)
   <223> Primer lysEK-1
<400> 11
   tgcttgcaca aggacttcac 20
<210> 12
   <211> 20
   <212> DNA
   <213> Corynebacterium glutamicum
<220>
   <221> misc_feature
   <222> (1)..(20)
   <223> Primer lysEK-2
<400> 12
   tatggtccgc aagctcaatg 20
<210> 13
   <211> 20
   <212> DNA
   <213> Corynebacterium glutamicum
<220>
   <221> misc_feature
   <222> (1)..(20)
   <223> Primer zwa1-A2
<400> 13
   cacttgtcct caccactttc 20
<210> 14
   <211> 20
   <212> DNA
   <213> Corynebacterium glutamicum
<220>
   <221> misc_feature
   <222> (1)..(20)
   <223> Primer zwa1-E1
<400> 14
   ttctactggg cgtactttcg 20